# EUROPEAN PATENT APPLICATION

(11) **EP 0 872 555 A1**
(43) Date of publication of application: **21.10.1998**
(21) Application number: 96925994.4
(22) Date of filing: 05.08.1996
(51) Int. Cl.: C12N 15/62, C12N 15/31, C12Q 1/02, C12N 1/21, C07K 19/00

(54) **BACTERIAL PEPTIDE LIBRARY**

(30) Priority: 04.08.1995 JP 199745/95
(71) Applicant: SUMITOMO ELECTRIC INDUSTRIES, LTD, Osaka-shi, Osaka-fu 541 (JP)
(72) Inventor: SHIMBARA, Naoki, Sumitomo Electric Ind., Ltd., Yokohama-shi, Kanagawa 244 (JP); SAYA, Hideyuki, Kumamoto-shi, Kumamoto 862 (JP)
(74) Representative: Baldock, Sharon Claire
(86) International application number: JP9602196
(87) International publication number: WO9706264

(57) **Abstract**

The present invention relates to a fusion protein specifically recognizing a particular target cell or protein. The fusion protein comprises a protein derived from a cell invasive protein (support protein) to be a support and a protein to be an object (object protein). This invwntion also relates to a bacterium presenting the fusion protein on its surface, and to a bacterial peptide library as a collection of the bacteria. The present invention permits to add desirable biological activities, such as binding ability to cells or proteins, invading ability into cells, to the fusion protein. Also, the bacteria of the present invention can be easily self-reproducible and is stable against any handling without degradation.

## Description

### Technical Field

This invention relates to a bacterial peptide library, and more specifically, to a method for constructing the library and a method for utilizing the same. This invention also relates to a bacterium or an object protein obtained by use of the bacterial peptide library.

### Background Art

Almost all biological reactions, such as immunological recognition, cell signalling and transmission, transcription and translation, are regulated by the recognition or binding of ligands. Of substances which serve as ligands, immuno-cytokines, growth factors, neurotransmitters and hormones are known to be mostly composed of proteins.

Various diseases may be grasped as abnormalities of the above immunological reactions, namely, abnormalities of ligand recognition or ligand binding, thereby attempts have been made to diagnose diseases by detecting these abnormalities, or to treat diseases by removing these abnormalities.

In diagnosing various pathological states, and establishing methods of treating them, it is also known to be important to recognize particular cells which change based on the pathological states. Thus, techniques for simple screening for the presence of particular cells emerging from particular disease states have been desired.

In the mechanism of recognizing particular cells, the ligand-specific recognition of the amino acid sequence of a receptor comprising a particular protein expressed mainly on the cell surface is also known to be an important step.

A known screening technique for many protein clusters is to construct by use of a phage a collection of peptides with a constant number of amino acid residues that can be arranged in a random sequence (a random peptide library) and to express the target peptide on the surface of the phage (Science, 249, 386-390, 1990). This technique, however, is not suitable for use in experiments on invasion into particular cells, and involves the problem of cost, because the phage peptide library needs to be re-transmitted to a host, such as E. coli, in growing clones.

The expression of a fusion protein using the outer membrane protein of E. coli has been tried (Gene, 70, 181, 1988). With this method, conditions for stably expressing the protein have not been established. Thus, efficient presentation of the protein on the surface of E. coli is difficult, making it questionable to put the method into practical use.

In conclusion, technologies for simple screening for the presence of a particular cell emerging from a particular disease state and for providing a therapeutic agent and method based on the results have not been established. Development of such technologies has been in earnest demand.

Recently, it has been proposed that a thioredoxin random peptide library is expressed on the surface of E. coli by use of the flagellum protein of E. coli (Biotechnology vol 13, April 1995, pp.336 - 372).

### Disclosure of Invention

The object of the present invention is to provide a bacterium and a bacterial peptide library which express an object protein on the surface of the bacterial cell(s). The object of the present invention is to provide a bacterium and a bacterial peptide library which express an object protein having any amino acid sequence on the surface of the bacterial cell(s). The object of the present invention is to provide a novel screening system by utilizing particular ligand recognition.

We have thought that the needs will be fulfilled by constructing a novel bacterium and a novel bacterial library which are multipliable efficiently and stably, which are relatively resistant to change or deterioration during storage, which can specifically recognize a particular cell or protein, which can, if desired, show desirable biochemical activities, and which are free to include other biological activities additionally.

Based on the above concept, we have considered that it is desire to provide a novel bacterium, and more concretely, that type of a well known bacterium such as E. coli, namely, a self-reproducible bacterium which has an active site, such as a recognition site, on the surface of the cell in order to eliminate the need for a cell degradation step or the like, and whose active site (such as recognition site) comprises at least a protein derived from a cell invasive protein and an object protein, the object protein recognizing a receptor (a receptor protein on the surface of a target cell, such as , particular receptor including a cytokine receptor, a growth factor receptor, a hormone receptor and an invasion-receptor, an antibody and a growth factor). These requirements would permit the production of a bacterium which can specifically recognize a particular cell or protein, and the production of a bacterial peptide library including the bacterium.

This invention provides a fusion protein, which protein is presented on the surface of bacterial cells, comprising at least a protein derived from cell invasive protein and an object protein, an bacterium including a desired biochemical activity introduced in its active site (such as recognition site), and a bacterial peptide library including the bacterium.

On the basis of the above consideration, we have made an extensive study, and found that a novel bacterium satisfying the above-described requirements can be constructed by using a plasmid vector incorporating DNA encoding the fusion protein.

We have also made an extensive study on how to present a protein, having biological activity, on the surface of a bacterium efficiently and stably. This study has led us to find a method for expressing a fusion protein, comprising a protein as an object (object protein) fused to a protein derived from a cell invasion protein, on the surface of a bacterium; to develop a novel bacterium having the fusion protein expressed, namely, a fusion protein surface-presented bacterium; and to discover the way of constructing a library of such bacteria.

We have further found the way of constructing a bacterium expressing a random peptide as the object protein, thereby a bacterial peptide library as a collection of such bacteria has been made.

We have further found a novel screening technique using the above-mentioned bacterial peptide library. That is, we have found that a method of screening for a protein having biological activity can be provided using this library. For example, we have found that a method of using the library is useful in elucidating the amino acid sequence of a peptide having an invasion signal for cell invasion, or in clarifying the amino acid sequence of the active site of an enzyme or the combining site of a protein such as epitope of an antibody.

We have also found that the above bacterial peptide library is useful for a method of diagnosis, a therapeutic agent, and a treating method for a specific disease.

### Brief Description of Drawings

Fig. 1 is a schematic view showing the construction of a plasmid having a BstX I linker inserted into a gene encoding the protein derived from a cell invasive protein, part of invasin, relevant to the present invention; and
Fig. 2 is a view showing the production of a random oligonucleotide relevant to the present invention.
Fig. 3 is a view showing the binding ability of EL-ESPEL to a cell compared with that of the control clones after one, two or three times of binding experiments.

### Best Mode for Carrying Out the Invention

A feature of the present invention concerns a fusion protein at least comprising a protein derived from a cell invasive protein and an object protein characterized in that at least a part of the fusion protein is presented on the surface of bacterial cells.

Here, the cell invasive protein is a protein which has cell invasion ability, which is expressed on the surface of bacterial cells, and which contributes to the invasion process based on the recognition of a target cell. This invasive protein includes a portion acting as an anchor to cell membrane. The protein derived from a cell invasive protein used in the present invention is a protein derived from such cell invasion protein described above, and a protein acting as a support which is expressed on the bacterial surface to present an object protein on the bacterial surface when the protein is expressed in the bacterium of the present invention (hereinafter, the protein derived from a cell invasive protein acting as a support for an object protein simply refers to a support protein if it is convenient). The protein derived from a cell invasive protein used in this invention may be a part of the cell invasive protein, a derivative of the cell invasive protein, that is, a cell invasive protein having a variation in a part of itself or a protein derived from a cell invasion protein having a variation in a part of itself, or a cell invasion protein itself as long as such proteins can act as a support for the above requirement, but preferably is a part of or a variant of a cell invasive protein which has lost its ability of invading cells.

The object protein used in this invention refers to a protein expressed on the protein derived from a cell invasive protein as the above support protein and presented on the bacterial surface. The object protein is not limited as long as it fulfills the above requirements, and in accordance with a purpose for use, any protein having any feature may be the object protein used in this invention. For example, the object protein includes a ligand capable of binding to a receptor, a invasion signal peptide or protein contributing to the process of invading into cells, or many and unspecified random peptides for use in screening. Therefore, the presentation of at least a part of the fusion protein on the surface of bacterial cells in the present invention means that the above fusion protein, preferably the object protein is held on the surface of bacterial cells via the protein derived from a cell invasive protein as the support so that the properties of the fusion protein (preferably object protein) can be expressed. For instance, an action is meant, such that the object protein is held on the surface of bacterial cells via the support protein so as to take on a specified steric structure.

Another feature of the present invention relates a bacterial surface-presented fusion protein, at least a part of which is presented on the surface of bacterial cells, characterized in that the fusion protein comprising a protein derived from a cell invasive protein and an object protein. The fusion protein comprising a protein derived from a cell invasive protein and an object protein used in the present invention means an object protein combined with a protein derived from a cell invasive protein or an object protein inserted into any portion of a protein derived from a cell invasive protein as long as the fusion protein is presented on the surface of bacterial cells.

The other feature of the present invention is a bacterial surface-presented fusion protein, at least a part of which is presented on the surface of bacterial cells, characterized in that the fusion protein comprising a protein derived from a cell invasive protein and an object protein combined with C-terminal portion of the protein derived from a cell invasive protein. The combination with the C-terminal portion of the protein includes the way in which the object protein is combined with the C-terminal directly and the way in which any amino acid sequence is an intermediate between the object protein and the C-terminal. The above any amino acid sequence is not limited and includes a spacer sequence described thereinafer as well as any sequence required to fit a frame and the like to a desired condition in gene engineering procedure.

Furthermore, the other feature of the present invention relates to the fusion protein containing at least one spacer. Here, the spacer refers to a peptide or amino acids having a structure which, if desired, can be cut at least with a specified enzyme, or enable the object protein to be presented stably on the surface of bacterial cells.

The other feature of the present invention is also related to the fusion protein in which the support protein is part of a cell invasive protein.

Also the other feature of the present invention is that the above cell invasive protein of the fusion protein is selected from the group consisting of invasin or ail of Yersinia enterocolitica, invasin or ail Yersinia Enterocolitia, ipa B, ipa C or ipa D of Shigella flexneri, inv A, B, C, D, H or hil of Salmonella typhimurium, eae or cfm of pathogenic Escherichia coli, and a cell invasive protein presented on the surface of Leishmania protozoan.

Moreover, the other feature of the invention relates to the fusion protein in which the cell invasive protein is invasin having an amino acid sequence described as Seq. ID No. 1 in the Sequence Listing.

The feature of the present invention also relates to the fusion protein in which the support protein of the fusion protein is invasin having an amino acid sequence described as Seq. ID No. 1 in the Sequence Listing, the invasin excluding at least cell invasion signal protein or peptide, preferably, relates to the fusion protein in which the support protein is an amino acid sequence described as Seq. ID No. 2 in the Sequence Listing.

The other feature of the present invention also relates to the fusion protein in which the support protein of the fusion protein is modified. Here, the modification or variant of the support protein refers to a protein in which an amino acid has been substituted, deleted or added artificially or spontaneously, with the function of the support protein being substantially retained.

A feature of the present invention also relates to the fusion protein in which the object protein of the fusion protein has a ligand effect on a receptor. Here, the ligand effect refers to capability of binding the receptor. Examples of the receptor are receptor proteins on the surface of a cell, antibodies, and growth factors, and further include polynucleotides such as DNA and RNA.

Also, the other feature of the present invention relates to a method in which DNA encoding a fusion protein comprising a protein derived from a cell invasive protein and an object protein (wherein the DNA includes at least DNA sequence encoding the protein derived from a cell invasive protein and DNA sequence encoding the object protein) is inserted into a vector, the vector is transferred into a bacteria to be transformed, the fusion protein is expressed in the bacteria so that the fusion protein is presented on the surface of bacterial cells.

The other feature of the present invention relates to a method in which DNA encoding a fusion protein comprising a protein derived from a cell invasive protein and an object protein (wherein the DNA includes at least DNA sequence encoding the protein derived from a cell invasive protein and DNA sequence encoding the object protein located on the 3'-portion thereof) is inserted into a vector, the vector is transferred into a bacteria to be transformed, the fusion protein is expressed in the bacteria so that the fusion protein is presented on the surface of bacterial cells.

Also, a feature of the present invention relates to a method for presenting the fusion protein on the surface of bacterial cells in which the above DNA encoding the fusion protein further includes a DNA sequence encoding a spacer consisting of amino acids.

Furthermore, a feature of the present invention relates to a method for presenting the fusion protein on the surface of bacterial cells characterized in that the above DNA sequence encoding a protein derived from a cell invasive protein is a DNA sequence encoding a protein derived from invasin.

Also, the other feature of the present invention is a method for presenting the fusion protein on the surface of bacterial cells in which the above DNA sequence encoding a protein derived from a cell invasive protein is the DNA sequence described as Seq. ID No. 3 in the Sequence Listing and the DNA sequence encoding the object protein is random.

Also, the other feature of the present invention is a method for presenting the fusion protein on the surface of bacterial cells in which the object protein of the fusion protein is random peptide, preferably random peptide having 3 - 16 amino acids.

A feature of the present invention also relates to a method for presenting the fusion protein on the surface of bacterial cells in which the above DNA sequence encoding the object protein is DNA sequence of the formula (NNK)ₙ where N is one of deoxyribonucleotides being A, G, C and T, K is one of deoxyribonucleotides being G and T.

In the above formula, n preferably denotes an integer of 3 to 16.

A feature of the present invention relates to a method for preparing an object protein and which method includes the following steps:
(a) inserting DNA encoding a fusion protein into a vector, which fusion protein comprises a protein derived from a cell invasion protein, an object protein and a spacer, consisting of amino acids having enzyme digestion site, located between both proteins,
(b) introducing the vector into bacterial cells to be transformed and presenting the fusion protein on the surface of bacterial cells,
(c) multiplying the transformants,
(d) digesting the spacer of the fusion protein presented on the bacterial surface by use of an enzyme,
(e) isolating the object protein cut off from the bacteria.

A feature of the present invention relates to a method for preparing an object protein, in which the above DNA encoding the fusion protein comprises a protein derived from a cell invasive protein, an object protein and a spacer, consisting of amino acids having an enzymatic cleavage recognition site, located between both proteins, is characterized in that the DNA sequence encoding the protein derived from a cell invasive protein is located on the 5'-side of the DNA sequence encoding the spacer and the DNA sequence encoding the spacer is located on the 5'-side of the DNA sequence encoding the object protein.

The other feature of the present invention relates to a method for preparing an object protein in which the method further includes the following step after the step (c);

### (c-1) screening the transformant presenting a desired fusion protein on the surface of bacterial cells.

Also a feature of the present invention relates to a DNA encoding the above fusion protein.

A feature of the invention further relates to a DNA comprising a base sequence of the formula (NNK)ₙ where N is one of deoxyribonucleotides being A, G, C and T, K is one of deoxyribonucleotides being G and T, and n donates an integer of 3 to 16, joined to a base sequence described as Seq. ID No. 3 in the Sequence Listing.

The invention also relates to the above DNA with n being 10.

The present invention also relates to a fusion protein surface-presented bacterium presenting at least object protein of the fusion protein on the surfaces of bacterial cells. The invention further relates to the fusion protein surface-presented bacterium characterized in that the bacterial cells are gram-negative bacterial cells, and more particular, E. coli cells. A feature of the present invention relates to a bacterial peptide library as a collection of the above fusion protein surface-presented bacterium.

A feature of the present invention relates to the bacterial peptide library as the collection of the bacterium expressing the fusion protein on its surface, in which fusion protein the protein derived from a cell invasive protein is a protein derived from invasin and the object protein comprising random peptides with 3 - 16 animo acids.

A feature of the present invention also relates to the fusion protein surface-presented bacterium in which the fusion protein has a ligand effect on a receptor. Also the present invention relates to the fusion protein surface-presented bacterium in which the fusion protein has ability of invading into cells. A feature of the present invention further relates to the fusion protein surface-presented bacterium in which the fusion protein has ability of binding cells. Also the present invention relates to the fusion protein surface-presented bacterium in which the fusion protein has ability of invading into cells and a ligand effect on a receptor.

The present invention also relates to a method for identifying a protein having biological activity by the bacterial peptide library, and a novel screening system utilizing the bacterial peptide library. The above biological activity may be any biological activity held by the fusion protein or the object protein presented on the surface of bacterial cells, for example, includes a ligand effect on a receptor, ability of binding cells and ability of invading cells.

The other feature of the present invention relates to a protein having a biological activity identified by utilizing the bacterial peptide library of this invention, such as a recognition signal of particular proteins or cells or invasion signal for invading into particular cells. The other feature of the present invention further relates to DNA encoding the identified protein having biological activity.

The further feature of the present invention relates to a diagnosis method utilizing the bacterium having particular properties described above. A fusion protein or an object protein, specifically recognizing cancer cells, or the object protein selected by use of the screening method of this invention, or a bacterium having a fusion protein or an object protein is useful for diagnosing cancer. Furthermore, the present invention relates to a diagnostic agent or diagnostic kit using a bacterium or at least part of a fusion protein, preferably an object protein selected by use of the screening system of this invention, and to a diagnosis method using such diagnostic agent or kit.

A feature of the present invention relates to a therapeutic agent for disease, comprising at least part of the fusion protein, preferably, the object protein, and a therapeutic substance. Also, a feature of the present invention relates to a therapeutic agent for disease, comprising the fusion protein preferably, the object protein as a therapeutic agent screened by use of the method of this invention,. Also a feature of the present invention relates to a therapeutic method for specifically healing a cell with the disease by use of the therapeutic agent for disease.

A feature of the present invention also relates to a recombinant vector containing a DNA encoding the fusion protein, and the recombinant vector in which the vector is a plasmid vector.

In the instant specification and drawings, the abbreviations according to the IUPAC-IUB or the abbreviations in customary use among those skilled in the art are used when bases or amino acid are expressed by abbreviations. Amino acids are described in three letters or one letter.

### Nucleic acids

- DNA: Deoxyribonucleic acid
- A: Adenine
- C: Cytosine
- G: Guanine
- T: Thymine

### Amino acids

- Ala (A): Alanine
- Arg (R): Arginine
- Asn (N): Asparagine
- Asp (D): Aspartic acid
- Cys (C): Cysteine
- Gln (Q): Glutamine
- Glu (E): Glutamic acid
- Gly (G): Glycine
- His (H): Histidine
- Ile (I): Isoleucine
- Leu (L): Leucine
- Lys (K): Lysine
- Met (M): Methionine
- Phe (F): Phenylalanine
- Pro (P): Proline
- Ser (S): Serine
- Thr (T): Threonine
- Trp (W): Tryptophan
- Tyr (Y): Tyrosine
- Val (V): Valine

### (Target cell or protein the invention is directed to)

The present invention relates to a fusion protein capable of specifically recognizing a particular target cell, protein or various compositions or mixtures including protein as a part of themselves, and a bacterium presenting the fusion protein on its surface.

The present invention relates to a bacterial peptide library for screening and preparing a fusion protein including an object protein capable of specifically recognizing a particular target cell or protein.

Thus, the recognition of the target by the invented bacterium is presumed to be ascribed mainly to an interaction, based on the recognition of a particular amino acid sequence, between a fusion protein presented on the bacterial surface and the target cell or protein. This means that a cell to become the target in the present invention is not limited as long as it has an amino acid sequence, which makes the cell specifiable, presented in the vicinity of the cell surface. Also the protein to become the target in the present invention is not limited as long as it has an amino acid sequence making the protein specifiable.

Furthermore, the above-described recognition based on the amino acid sequence of the target is not necessarily restricted to the one attributed to a direct interaction, but includes the one ascribable to complex interactions. The recognition portion to be presented near the surface of the cell to be the target of the present invention includes not only an amino acid sequence inherent in the cell, but also an amino acid sequence to be introduced by a suitable artificial means or in a spontaneous fashion.

Thus, the kinds of the target cell to be recognized in the present invention are not restricted as long as they have the foregoing properties. For example, human or various other animal cells may be used as the target cell to be recognized in this invention. The various animal cells can be obtained from an organ such as the liver, kidney, heart, brain, lung, thymus or pancreas. The cell to become the target need not be a normal cell. Cells emanating from various diseases may be used preferably. As the diseased cells, cells, for instance, from cancer, AIDS, or autoimmune disease may be used without restriction, as long as they have the features described above. The fusion protein, the object protein or the bacterium of this invention which can recognize a cell derived from particular disease can be obtained by using the bacterial peptide library of this invention, and such proteins or bacterium are useful for diagnosing and treating the disease.

As a target to be recognized in this invention, any protein may be used. For example, in case that particular receptor is used as a target, a fusion protein, an object protein or a bacterium of this invention which specifically recognize the receptor can be obtained by using the bacterial peptide library of this invention. Furthermore, a fusion protein, an object protein or a bacterium of this invention which recognize particular protein derived from particular disease can be obtained by using the bacterial peptide library of this invention, and such proteins and bacterium are useful for diagnosing and treating the disease.

### (Fusion protein surface-presented bacterium and bacterial peptide library)

The fusion protein surface-presented bacterium of the present invention refers to a bacterium which have expressed on or near its surface a fusion protein comprising a protein derived from a cell invasive protein (support protein), and an object protein fused thereto. The bacterial peptide library of the present invention refers to a collection of the above bacteria, preferably the bacteria presenting an object protein having 3 - 16 amino acids. Here, to be presented on or near the surface of the bacteria includes the case that the fusion protein of the present invention actually exists on the surface of the bacteria, and the case that only a part of the fusion protein exists on or near a part of the surface of the bacteria as long as the fusion protein (preferably the object protein) can virtually recognize a target cell or protein (in the present invention, the term of to be presented on the surface of the bacteria refers to these cases).

No restriction is imposed on usable bacterial in the present invention. The bacteria include those selectable by people skilled in the art according to some exemplifying to be described below. That is, the bacterial related to the present invention may be those transformable by a suitable vector or the like so that at least the fusion protein of the present invention can be presented on or near the surface of the bacteria. The transformed bacterial need to be those which can be multiplied by known ordinary means.

Hence, various bacteria can be used preferably in the present invention. For example, staphylococci and streptococci are usable as gram-positive bacteria. Gram-negative bacteria are particularly preferred. For instance, bacteria of Enterobacteriaceae, Vibrionaceae and Pasteurellaceae are usable. Of the gram-negative bacteria, Escherichia coli of Enterobacteriaceae can be used preferably, as disclosed in detail in the Examples in the specification of the present application.

As explained above, the fusion protein, the bacterium presenting the fusion protein, and the bacterial peptide library as a collection of the bacteria of the present invention recognizes, based on a direct or indirect interaction, a particular amino acid or a particular target protein presented near the surface of the target cell described above. If the fusion protein or the bacterium of the present invention has the ability of invading a target cell, it can invade the cell to be recognized by itself. Also, if the fusion protein or the bacterium of the present invention has the ability of binding a target cell, it can bind the cell to be recognized by itself.

### (Protein derived from cell invasive protein, object protein, spacer)

In the present invention, the protein derived from a cell invasive protein is a protein which is derived from any cell invasive protein existing in various bacteria, and a protein which is appeared on the surface of bacterial cells and acts as a support for presenting an object protein on the surface of bacterial cells when it is expressed in the bacteria of the present invention. The protein derived from a cell invasive protein used in the present invention may be, as long as it can act as the support described above, a part of the cell invasive protein, a modification or variant of the cell invasive protein, namely modified cell invasive protein or modified a part of cell invasive protein, or the cell invasive protein itself, preferably a part of the cell invasive protein losing the ability of invading cells or its variant.

Here, the cell invasive protein refers to a protein which exists in bacteria having the ability of invading into animal cells, which is expressed on the surface of the bacteria and which has the ability of invading into cells. Of the gram-negative bacteria, a bacterium known of having pathogenicity is known to have the ability of invading into animal cells such as epithelial cells or macrophages. The mechanism for these bacteria to invade into animal cells is presumed that such bacteria present a particular protein for invading into animal cells (which protein refers to the cell invasive protein) and the animal cells recognize the protein and then the bacteria invade into the animal cells (BioEssay, 15, 17-24, 1993 ; Current Opinion in Immunology 1994, 6, pp.590-595 ; Current Opinion in Immunology 1995, 7, pp.479-484, all of which are incorporated herein by reference). As such cell invasive protein includes the following proteins.

An example of the cell invasive protein is invasin (inv), a cell invasive protein expressed while Yersinia pseudotuberculosis, a gram-negative bacterium, is invading a cell (Cell, vol., 50, 769-778, August 28, 1987; its amino acid sequence is described as Seq. ID No. 1 in the Sequence Listing). Other examples include the inv of Yersinia enterocoliticia (Mol. Microbiol., 4, 1119-11128, 1990) and ail (attachment invasin locus) protein of Yersinia enterocoliticia (J. Bacteriol., 172, 1062-1069, 1990), ipa B, ipa C and ipa D of Shigella flexneri (Rev. Infect. Dis., 13, suppl. 14, 285-292, 1991), inv A, B, C, D and H of Salmonella typhimurium (Pro. Natl. Acad. Sci., USA, 86, 6383-6387, 1989) and hil of Salmonella typhimurium (Pro. Natl. Acad. Sci., USA, 89, 1847-1851, 1992), eae and cfm of enteropathogenic Escherichia coli (EPEC) (Infect. Immun., 58, 1565-1571, 1990), and a cell invasive protein presented on the surface of leishmania. Hereinafer, these cell invasive proteins refers to the invasin family if it is convenient. All of the above articles are incorporated herein by reference.

In the present invention, the protein derived from a cell invasive protein can be used with satisfaction. Of the above invasin family, any invasin, such as inv of pseudotuberculosis, inv of enterocolitica,and inv A, B, C, D and H of Salmonella typhimurium can preferably be used, and the protein derived from the invasin of Yersinia pseudotuberculosis, in particular, is preferably used in the present invention.

When the support protein in the present invention is the above cell invasive protein itself, it is not suitable for the purpose of recognizing a particular protein or particular cell because of the cell invasive protein inherently having the ability of recognizing animal cells and invading into them. In such case, instead of the above cell invasive protein itself, part of the above cell invasive protein or the above cell invasive protein itself having any variation as to lose its ability of invading cells, such as those proteins in which amino acid(s) are substituted, deleted therefrom or added thereto and any variants of those proteins, can be used. As the support protein of the present invention, a protein in which any peptide is added to any part of the above cell invasive protein (for example, after the invasion signal of the cell invasive protein) can be used, the peptide may be any amino acid sequence unless it damages the properties of presenting the object protein on the surface of bacterial cells, moreover, may be the object protein itself.

The preferable embodiment of the present invention is that the protein derived from a cell invasive protein has lost its ability of invading cells which ability the cell invasive protein inherently had, and has the amino acid sequence excluding the cell recognition region of the cell invasive protein. The more preferable embodiment is that as the object protein, a random peptide is combined with the protein derived from a cell invasive protein directly or by using a spacer as an intermediator, or that as the object protein, a random peptide is inserted into the protein derived from a cell invasive protein directly or by using a spacer as a mediator.

The rule of the protein derived from a cell invasive protein of the present invention is that of the support protein presenting the object protein on the surface of bacterial cells. For the rule, the protein derived from a cell invasive protein of the present invention preferably maintains the amino acid sequence contributing to its anchoring to bacterial membrane.

If the amino acid sequence of the protein derived from a cell invasive protein is known, for instance, those skilled in the art can specify the object protein and prepare the fusion protein comprising the protein derived from a cell invasion protein and the object protein by known biochemical means. A feasible example is to decide on DNA encoding the cell invasive protein, obtain a portion containing DNA encoding an amino acid sequence which recognizes a particular receptor of a cell during invasion into the cell (invasion signal peptide), remove this portion by means of a restriction enzyme or the like to make DNA encoding the support protein, and join DNA, encoding the aforementioned random peptide, to the remaining portion with a ligase or the like, thereby preparing DNA encoding the fusion protein. When this DNA is introduced into a suitable vector to transform a suitable bacterium, a fusion protein having the support protein and the object protein can be expressed on the bacterial surface.

The fusion protein of the present invention, moreover, can incorporate some spacer amino acids or spacer peptides, if desired. The incorporation of the spacers is intended, partly, to support the object protein, expressed on the support protein, more stably on the bacterial surface, and partly to cut off the spacer region enzymatically if desired, to make only the object protein part separable. To attain the first purpose, proline (or including oligoproline) can be suitably selected as an amino acid. Since proline is limited in the degree of steric freedom, it can be used preferably to maintain the object protein with a constant structure. If a plurality of prolines are arranged for use as the spacer, their suitable number is 3 to 10. A peptide having a specific high-order peptide structure (ZZ peptide of 14 kDa long originating from B domain of protein A) may also be used (Method Enzymol., 198, 3, 1991, which is incorporated herein by reference). In this case, the object protein can be presented stably on the surface by maintaining the specific high-order structure.

In regard to the second purpose, it is easy for those skilled in the art to choose a suitable amino acid sequence and the number of the suitable amino acids, on the basis of the characteristics of the cleavage enzyme selected beforehand. To attain this, a known method can be referred to. Thus, it is possible to provide the spacer at an arbitrary site, such as at a suitable position of the support protein, or between the support protein and the object protein. Either of the preferred amino acids, the preferred number of the amino acids, or the preferred site where the spacer is inserted can be selected with free, and the method of inserting the spacer may follow known biochemical means. That is, the DNA encoding the desired amino acid sequence and the desired number of amino acids can be inserted into any site by use of a known gene engineering technique. If the spacer is cleaved by thrombin, DNA (CTG-GTT-CCG-CGT-GGA-TCC) encoding (Leu-Val-Pro-Arg-Gly-Ser) is introduced into a vector, whereby the peptide is expressed and can be cleaved by thrombin. Likewise, if Factor Xa is used for cleavage, DNA (ATC-GAA-GGT-CGT-YYY where YYY is DNA not encoding Arg or Pro) encoding (Ile-Glu-Gly-Arg-X where X is an amino acid other than Arg or Pro) may be inserted into a vector (Method in Molecular and Cellular Biology, 4, 220-229, 1993, which is incorporated herein by reference).

### (Object protein)

The object protein described previously, which is a part of the fusion protein, can be selected without restriction in the present invention. The object protein of the present invention can recognize the target cell or protein mainly basis of protein-protein interaction. Thus, there is no restriction on the object protein, as long as it can fully recognize the target cell or protein by the interaction.

One of feasible and preferable embodiments in the present invention is to use as the object protein a random peptide having a randomly selected consecutive amino acid sequence in order to screen and identify the object protein having a particular amino acid sequence showing a specific interaction with the particular target. For example, combinations of 10 randomly selected amino acids from 20 kinds of amino acids are available theoretically in about 1 x 10¹³ kinds (about 1 trillion kinds), a number enough large to detect a particular amino acid sequence by screening.

There is no restriction on the number of amino acids to be randomly selected for the object protein. The number of the amino acids does not pose a technical constraint to those skilled in the art in working out the bacterial peptide library. The use of known synthesizing means facilitates the introduction of any desired number of amino acids, preferably 3 to 16 random peptides. For instance, DNA encoding the random peptides can be synthesized by a commercially available DNA synthesizer.

In presenting a random peptide as an example of the object protein in an embodiment of the present invention, DNA encoding the random peptide (random DNA) can be expressed preferably by (NNK)ₙ where N is a deoxyribonucleotide being A, G, C or T, and K is a deoxyribonucleotide being G or T.

n denotes the number of amino acids constituting the random portion (Experimental Medicine, vol. 11, No. 13, 1993, which is incorporated herein by reference).

When n = 1, (NNK)₁ is any of 32 possible DNA's shown in the Table 1. n is at least 3, and preferably, a number encoding 6 to 15 amino acids. It goes without saying that the number for n is not restricted by the method for synthesizing the random DNA, but depends on the properties of the target cell to which the library of the present invention is to be applied. Thus, there is virtually no restriction on the number for n other than n being at least 3 in the present invention.

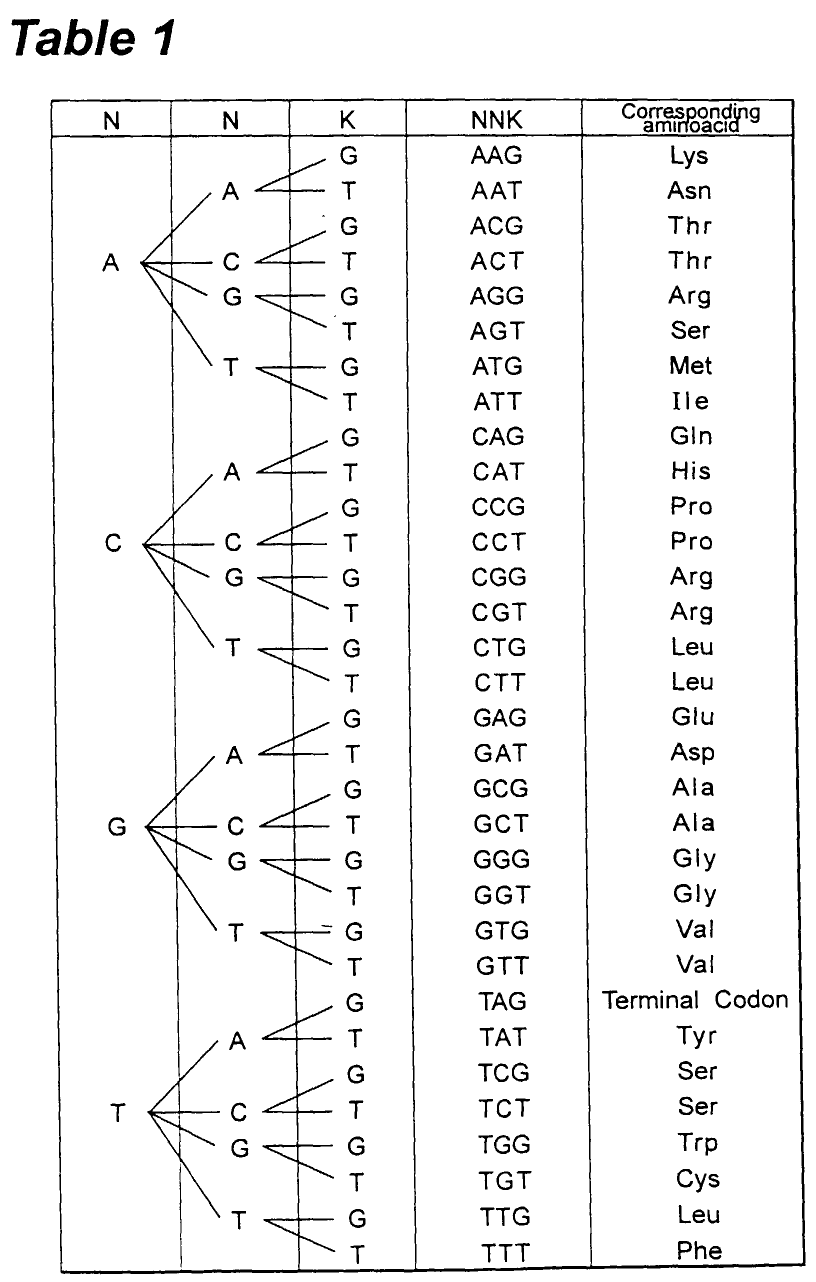

The object protein can be presented, with high efficiency, by adding cysteine to both ends (N-terminal portion and C-terminal portion) of the object protein. The N-terminal portion of the object protein means the N-teminus of the object protein as well as the portion within several or dozens of amino acids from the N-teminus, and the C-terminal portion of the object protein means the C-teminus of the object protein as well as the portion within several or dozens of amino acids from the C-teminus. The high efficiency means to lessen the effect of steric hindrance on the reactivity of the object protein. If a disulfide bond is formed between the SH groups of cysteines added to the opposite ends, the object protein will be presented, with a looped structure, on the surface of the fusion protein. Thus, the influence of steric hindrance is lessened, and the object protein becomes more reactive. When the object protein is inserted between any two cysteins of the support protein and a disulfide bond is formed between the SH group of cysteins, the effect as same as that of the above example can be attained. The known molecules having a loop via cysteine includes members of the immunoglobulin family, such as immunoglobulins (IgG, IgM), T cell receptor, MHC Class II molecule, LFA-3, ICAM-1, and VCAM-1. The size of the object protein suitable for forming the loop can be designed by referring to these known molecules. To separate and recover the object protein after screening, a known biological method can be applied. Although no restriction is imposed, in the present invention, the object protein becomes preferably separable by providing a suitable spacer amino acid sequence between the support protein and the object protein as described previously.

### (DNA encoding fusion protein)

The DNA encoding the fusion protein of the present invention is the DNA comprising the DNA encoding the protein derived from a cell invasive protein and the DAN encoding the object protein. The DNA may include a DNA encoding a spacer or any amino acids, at any position of the DNA, such as between the DNA encoding the protein derived from a cell invasive protein and the DNA encoding the object protein, as long as which does not effect the properties of presenting the fusion protein on the surface of bacterial cells.

DNA encoding the fusion protein with the aforementioned makeup includes a modified product, unless it loses the activity of the fusion protein surface-presented bacterium of the present invention. That is, any variant DNA can be used as long as the DNA fulfills the following requirements: the variant DNA, obtained by the spontaneous or artificial substitution or deletion of the base sequence of the DNA or insertion of a base sequence into the DNA, is introduced into a suitable vector to transform a suitable bacterium, and the resulting bacterium presents the fusion protein on its surface, the presented fusion protein has the support protein and the object protein. The fusion protein may, if desired, be those which can recognize the target cell and moreover invade the cell. Artificial preparation of the variant can be performed by people skilled in the art, if they use various known methods and make choices.

In a preferred embodiment of the present invention, the DNA encoding the fusion protein is those including the DNA encoding the protein derived from the invasin family proteins and the DNA encoding the object protein. In another preferred embodiment of the present invention, the DNA encoding the fusion protein is those including the DNA encoding the protein derived from the invasin family proteins and the DNA encoding the object protein and inserting the DNA encoding the object protein located in the 3' side of the DNA encoding the protein derived from the invasin family proteins. In the other preferred embodiment, the DNA is those encoding the fusion protein in which the object protein is a random peptide with 3 - 16 amino acids long.

### (Embodiment of construction of bacterial peptide library)

The constituent proteins of the bacterial peptide library as a collection of the bacteria having the fusion protein of the present invention have been described along with their properties. A suitable embodiment of the construction of the library will be described.

The bacterial peptide library of the invention adopts bacteria as a donor of a surface on which the fusion protein is presented. Thus, this library is expected to give some advantages not seen in a conventional surface random peptide library using phage or the like. Since the bacteria per se have a self-replicating ability, the fusion protein or the object protein, or the gene encoding them can be obtain easily and in large amounts by multiplying the bacteria having a part of the fusion protein expressed on the surface thereof. If the fusion protein capable of binding a receptor is presented on the surface of the bacteria, it is possible to elucidate the ligand effect on the receptor by use of the fusion protein. If the fusion protein presented on the surface of the bacteria has cell invasiveness, it is possible to elucidate the mechanism of invasion of bacteria or a substance into a cell, or to acquire a substance selectively invading a cell.

On the surface of the bacteria, the object protein with the same amino acid sequence is presumed to be presented in a larger amount than in the case of phage. This makes it easy to screen the object protein more efficiently. For example, when the object protein is assayed by absorbance using antibodies reactive with the object protein, the more the object protein is presented on the bacterial surface, the more antibodies react. Larger measurements are obtained, and the S/N ratio becomes better, producing more accurate assays.

There is no restriction on the usable bacteria in the present invention, as long as they have the above-described general properties. Staphylococci and streptococci are usable as gram-positive bacteria. Bacteria of Enterobacteriaceae, Vibrionaceae and Pasteurellaceae are usable as gram-negative bacteria. The use of Escherichia coli is preferred and possible, but the gram-negative species is not restricted to E. coli. Optimum bacteria may be chosen depending on the selection of the fusion protein to be used or the purpose of the screening. There is no restriction on selecting types of bacteria to be used when, for example, a protein derived from invasin is selected as the protein derived from a cell invasive protein. This is apparent by the fact that invasin can be expressed on other bacteria than Yersinia enterocolitica, as demonstrated by the fact that invasin from Yersinia enterocolitica was expressed on E. coli. Thus, embodiments of the present invention will be described with E. coli being as an example of the bacterium for use in the bacterial peptide library; however, other bacteria are similarly made available by employing conventional techniques.

A customary method for causing E. coli to express a protein comprises inserting DNA encoding the protein into a vector such as plasmid, introducing the vector into E. coli to prepare a transformant, and causing the transformant to express the protein. With this method, the intended protein is often expressed within E. coli cells, and it is difficult for the protein to be expressed in the vicinity of the bacterial cell surface. When the protein has been expressed within cells of E. coli, much labor and time are required for extracting the protein from the cells and purifying the extract. Thus, this method of merely using a transformant is not appropriate for the production of the object protein and its library.

The characteristic of the present invention lies in selecting the support protein for presenting the object protein in proximity to the bacterial cell surface in order to express the object protein, and in using DNA comprising DNA of the object protein joined to DNA of the support protein.

The vector for incorporating DNA encoding a fusion protein comprising the object protein fused to the support protein is not restricted, if the fusion protein can be presented on the surface of bacteria and the vector can be replicated and held in a host. Examples of the vector are plasmid vectors and virus vectors. Examples of the plasmid vectors include pBR322 (Gene, 2, 95, 1977), pBR325 (Gene, 4, 121, 1978; a derivative of pBR322), pUC12 (Gene, 19, 259, 1982), pUC13 (Gene, 19, 259, 1982), pUC18 (Gene, 33, 103, 1985), pUC19 (Gene, 33, 103, 1985), pUC118 (Method in Enzymology, 153, 3, 1987), pUC119 (Method in Enzymology, 153, 3, 1987), and BluescriptII (Nucleic Acids. Res., 17, 9494, 1989). Other plasmid vectors may be used, if they are replicated and held in hosts. Examples of the virus vectors include λgt10 (Huynh, T.V., Young, R.A. and Davis, R.W., DNA cloning, A Practical Approach, IRL Press, Oxford, 1, 49, 1985), λgt11 (Proc. Natl. Acad. Sci., U.S.A., 80, 1194, 1983) and λZAPII (Nucleic acids. Res., 17, 9494, 1989). Other vectors may be used, if they can grow in a suitable host. All of the above articles are incorporated herein by reference.

More preferably, the desirable vectors are plasmid vectors which are stable and easy to handle. The vector used in the Examples of the present invention is pBR325.

The method of incorporating the DNA into the vector may follow a known ordinary method.

The plasmid vector or virus vector incorporating the DNA can be introduced into a bacterium such as E. coli by a known ordinary method to transform the bacterium (Molecular Cloning, Cold Spring Harbor Laboratory Press, 1.3-1.72, 1989, which is incorporated herein by reference). As explained above, E. coli as a preferred host is for instance, HB101, JM109, CJ236, BL-21, DH5α and SURE2 can be used, if they can present the fusion protein substantially on the surface and allow screening of the fusion protein. In the Examples in the instant specification, embodiments using DH5α and SURE2 are disclosed in detail.

As the method of transforming a host with the plasmid vector, an ordinary known method, such as electroporation or the calcium chloride method described in Molecular Cloning, Cold Spring Harbor Laboratory Press, 1.74-1.84, 1989 (which is incorporated herein by reference), can be used preferably.

The virus vector can be introduced into multiplied E. coli by, say, in vitro packaging (Molecular Cloning, Cold Spring Harbor Laboratory Press, 2.3-2.118, 1989, which is incorporated herein by reference).

The resulting transformant is the fusion protein surface-presented bacterium. The Examples of the present invention involve a fusion protein expressed on the surface of E. coli cells, the fusion protein comprising the object protein fused to the support protein described as Seq. ID No. 2 in the Sequence Listing. The fusion protein is encoded either by DNA comprising DNA encoding the object protein joined to DNA described as Seq. ID No. 3 in the Sequence Listing; or by this DNA, if desired, also having, DNA encoding a suitable spacer joined thereto.

Furthermore, a collection of the fusion protein surface-presented bacteria expressing wide varieties of the object proteins, namely, a bacterial peptide library, can be constructed by preparing DNA's encoding wide varieties of the object proteins, inserting these DNA's into vectors, one DNA for one vector, and introducing the vectors into bacteria at a time.

In the present invention, moreover, at least one base of the base sequence of DNA can be substituted by other kind of base according to the degeneracy of genetic code, without changing the amino acid sequence of protein to be encoded by the DNA. Thus, the DNA of the present invention can also contain the base sequence converted by substitution based on the degeneracy of the genetic code. In this case, no variation occurs in an amino acid sequence induced from the base sequence obtained by the substitution.

The polynucleotide makes it possible to vary, spontaneously or artificially, part of the structure of a protein induced from the polynucleotide, without changing the principal activity of the protein. Of course, a protein encoded by the varied polynucleotide is a variant with substitution, deletion or insertion occurred in the amino acid sequence in comparison with the original protein.

Thus, the polynucleotide of the present invention contains a base sequence encoding polypeptides having structures corresponding to all protein variants described above.

With regard to the resulting fusion protein surface-presented bacterial peptide library, testing of whether the object proteins presented on the surfaces are peptides random enough to be expected or not can be done by, say, as follows: A suitable number of clones are sampled from part of the constructed library, and the base sequences of DNA's encoding the fusion proteins, or DNA's encoding the object proteins are determined by use of the clones. Then, the probability of appearance of each base A, T, G or C is compared with the expected value of appearance. The expected value of appearance can be calculated from the amounts of the respective bases used in the step of synthesizing DNA encoding the object proteins. The above base sequences can be determined by extracting plasmid from the library, and performing cycle sequencing using a suitable primer. The amino acid sequences of the object proteins are determined by base sequence information obtained. The base sequence information obtained by random sampling from the library is compared with the probabilities expected from DNA encoding the object proteins, such as the (NNK)ₙ, for testing purposes. This can be done by ordinary statistical analysis. Thus, the quality of the constructed library can be tested.

Another method is to isolate the amino acids of the fusion proteins presented on the surface of the library and determine the amino acid sequences. In detail, a suitable number of clones are grown from the library by random sampling, and the object proteins are cut and separated by suitable enzymes from the fusion proteins presented on the surfaces of the clones. For this purpose, there are used the fusion proteins having spacers provided in the vicinity of the object proteins so that the object proteins can be cut off by suitable enzymes. This procedure enables direct determination of the object proteins. That is, a separation membrane giving fractions of molecular weights suitable for the separation of the object proteins is used to purify only the object proteins. The resulting object proteins are put to a peptide sequencer, thereby determining the amino acid sequences. The randomness of the resulting amino acid sequences can be checked by an ordinary statistical analysis against the randomness of the amino acid sequences that has been expected.

The testing method described above can check how desirable the random peptides expressed by the resulting library are as the object proteins.

### (Introduction of additional biochemical activities)

The fusion protein, the bacterium presenting the fusion protein and the bacterial peptide library as a collection of the bacteria disclosed in the present invention is, if desired, introduced various other biochemical activities therein with no restriction of the above-mentioned properties, namely, the recognition of a particular cell or protein or any other substance as the target, and cell invasiveness optionally introduced. There is no restriction concerning whether these additional biochemical activities should be expressed on the surfaces of, or inside, the bacteria of the invention. Nor is there any restriction about whether these additional biological activities should function on the surface of the target or within the cells. The types of the additional biological activities that could be expressed are not restricted, either. For instance, they include cell growth, cell differentiation and cell adhesion.

Moreover, there is no restriction on the method for introducing the additional biological activity, an ordinary technique can be used.

The introduction of the additional biological activity can be attained, for example, by combining a therapeutic agent, such as anti-cancer agent or immunosuppressive agent with a part of the fusion protein, preferably the object protein of the present invention by use of an ordinary method utilizing covalent binding with chemical reaction or binding with a chemical crosslinking agent. Examples of the above therapeutic agents include anti-cancer agents such as cyclophosphamide, 5-fluorouracil and actinomycine, and immunosuppressive agents such as cyclosporine or its derivatives and azathioprine or its derivatives.

For example, it is possible to introduce a vector, incorporating DNA encoding a protein having biological activity or the like, into the fusion protein surface-presented bacterium by electroporation or the like, and to express the protein. Examples of the above protein having biological activity include various growth factors activating cells such as EGF and FGF, and various cytokines such as interleukine, interferon and tumor necrosis factor (TNF).

To express a particular physiologically active substance on vicinity of the surface of, or within, the target cell, DNAs encoding the physiologically active substance are incorporated into the bacteria of the present invention and after the bacteria recognize the target cell a substance acting on a promoter, which express the DNA, is added.

### (Screening based on cell binding or cell invasion)

By using the bacterial peptide library of the present invention, an object protein or a binding signal peptide contributing the bacterial binding to cells can be identified. If the fusion protein has cell binding ability, the library is reacted with the target cells on the basis of the cell binding ability of the fusion proteins relevant to the bacterial peptide library of the present invention, whereby only the bacteria bound to the target cells can be separated and recovered. By determining the amino acid sequences of the object proteins of the binding bacteria, it becomes possible to identify the binding signal for binding to the cells.

The screening method based on cell binding concerned with the present invention also discloses a separating method for separating the clone of the bacterium screened from the target cells.

Changes in the degree of binding dependent on differences in the cwll binding conditions are also considered specific of the target cell. Thus, highly selective screening by use of the library of the present invention becomes possible based on different binding conditions set.

The screening method based on cell binding related to the present invention is a method of screening the cell binding signal peptide as well as a signal peptide capable of invading cells. The fusion protein, the object protein or the bacterium having cell binding ability other than cell invasiveness can be screened by use of an ordinary technique, such as, labeling the fusion protein, the object protein or the bacterium screened based on the cell binding as described above with ,say, fluorescent substance, and observing the labeled protein or bacterium with fluorescent microscope.

Cells to become the target in the present invention are not restricted as have been described. Human or various other animal cells may be used. The various animal cells can be obtained from an organ such as the liver, kidney, heart, brain, lung, thymus or pancreas. Not only normal cells, but cells emanating from various diseases, such as cancer, AIDS, or autoimmune disease, may be used.

By using the bacterial peptide library it is possible to identify only an object protein or an invasion signal peptide contributing the cell invasion. If the fusion protein of the present invention has cell invasion ability, the library is reacted with the target cells on the basis of the cell invasiveness of the fusion proteins relevant to the bacterial peptide library of the present invention, whereby only those bacteria of the library invading the target cells can be separated and recovered. By determining the amino acid sequences of the object proteins of the invading bacteria, it becomes possible to identify the invasion signal peptide for invasion into the cells.

Thus, the screening method based on cell invasion concerned with the present invention discloses a novel method for screening based on the recognition of various interactions on the surface of the target cell, a hitherto well known method, and also screening based on whether the target cell is invaded or not.

Moreover, the invented screening method based on cell invasion discloses a method of separating the clones of the screened library from within the cell.

Changes in the degree of invasiveness dependent on differences in the cell invasion conditions are also considered specific of the target cell. Thus, highly selective screening by use of the library of the present invention becomes possible based on different invasion.

Cells to become the target in the present invention are not restricted as have been described. Human or various other animal cells may be used. The various animal cells can be obtained from an organ such as the liver, kidney, heart, brain, lung, thymus or pancreas. Not only normal cells, but cells emanating from various diseases, such as cancer, AIDS, or autoimmune disease, may be used.

An embodiment of screening based on cell binding to or cell invasion into a particular cell using the invented library will be described step by step.

The bacterial peptide library is prepared such that the number of the fusion protein surface-presented bacteria (clones), constituents of the library, will be suitable for the number of cells as the target. The cells and the library are reacted in a culture medium prepared so as to contain no antibiotic or serum, whereby the bacterium which has cell binding ability or cell invasion ability is caused to bind to or invade into the cells. The method of reaction for binding or invasion is not restricted, and known conditions can be selected. For instance, the cells should be reacted in as confluent a state as possible, because, in this state, there will be no nonspecific reaction with the culture dish or bottle. The number of clones of the library for use in the screening is not restricted, but when a 10 cm petri dish is used, there should be 10⁴ or more target cells in a confluent state.

The reaction time depends on the invasiveness of each clone of the library, but desirably is about 30 minutes to 4 hours. If only clones with high binding or invasiveness and invading in a short time are obtained, the reaction time may be short. If clones binding or invading only after a long time are obtained, however, the reaction time should be long. In the Examples of the instant specification, when human fibroblasts were used as the cells, reaction was performed for about 3 hours. Thus, the reaction time can be adjusted depending on the degree of invasiveness into the target cells, while changing the invasion conditions according to the purpose to be attained.

Elimination of nonspecific adsorption of each clone onto the cell surface is desirably performed by thorough washing with, say, phosphate buffer solution or culture medium. When the clone binding to or invading into cells is intended to be separated, the above washing is enough for the purpose, however, when the clone invading into cells is intended to be separated, an antibiotic against the bacterium used in preparing the library should be used.

The clones having invaded the cells can be extracted from within the cells. Various known means are usable. For example, surfactants may be used for extraction. According to embodiments of the present invention, 1% Triton-X100 can isolate the binding or invading clusters of clones from the cells without damaging E. coli. Thus, the above screening may be repeated if desired. For instance, it is desired to culture clusters of clones in antibiotic-containing LB medium or the like, and then perform cell binding or invasive experiments twice or more under the same conditions as described. These repeated experiments will increase the rate of clones with high binding ability or invasiveness. If clones with weak binding ability or invasiveness are desired, the experiments need not be repeated.

Separation, from the bacterial peptide library, of a single clone expressing on the surface a peptide having the desired binding or invasion signal can be done by an ordinary method. For example, the cells invaded by the clone are planted on LB plate containing an antibiotic such as ampicillin, and cultured, and then the colonies are separated.

To identify the signal peptide binding to or invading the cells, plasmid DNA is prepared from the binding or invading bacteria, and a primer of a suitable length, which permits sequencing of the region encoding the object protein, is synthesized. Then, the primer is subjected to the Maxiam-Gilbert method (Methods in Enzymology, 65, 499-560, 1980), the dideoxy method (Messing, J. et al., Nuclear Acids Research, 9, 309, 1981), or the Taq cycle sequencing method using fluorochrome (Biotechniques, 7, 494-499, 1989), whereby the base sequence can be determined. From this base sequence, the amino acid sequence of the object protein can be determined, whereby the binding or invasion signal peptide can be identified.

### (Screening based on ligand effect on receptor)

The use of the bacterial peptide library concerned with the present invention enables, for example, clones of the library having the binding fusion protein or object protein, which can specifically bind to the target cell or protein, to be screened and isolated based on the interaction with the target. For this purpose, various labeling of targets or the library by known methods can be used. After reacting the target with the library, the resulting target-library complex can be screened by using the label for detection. Since the binding of the library relies on a protein-protein interaction, examples of the target to be bound are generally various proteins, and may be various compositions or mixtures containing proteins. If other interactions than the protein-protein interaction are possible, the targets may be nucleic acids, and various compositions or mixtures containing nucleic acids. Moreover, cells presenting various proteins on their surface can preferably be used as the target. Proteins as targets include, for example, various antibodies, major histocompatibility antigens, receptors, ligands and synthetic peptides. Other proteins may be used, if they have the activity to bind to each clone of the library.

As the labeling method, FITC labeling, peroxidase labeling, radioisotope labeling (e.g., ³²P, ³⁵S or ¹²⁵I), or magnetic beads labeling is a preferred one capable of direct measurement. These preferred labeling methods can be practiced in various known ways. Methods capable of indirect measurement include one involving biotinylation. For detection, an avidin/biotin system is effective. For instance, an avidin-combined protein conjugated to peroxidase can be detected by reacting it with a biotinylated molecule.

The screening method of the present invention also permits the use of sandwich ELISA by using an antibody specifically reactive with the bacterial surface molecule of the clone of the library. The conditions for this method may follow known methods and techniques. For instance, streptavidin is coated on a plate or the like, and a biotinylated target protein (e.g. antibody) as a target for the surface molecule is immobilized on the coating. Then, the library is added to react with the target. Then, a peroxidase-conjugated anti-bacteria antibody is added to react only with the clone having reacted with the target protein, whereby color is developed. Thus, the reactivity of the clone of the library with the target protein can be examined quantitatively. (Therapeutic agent for disease, therapy for disease, diagnostic agent and diagnostic method using fusion protein, object protein or bacterial peptide library)

As described so far, screening by the bacterial peptide library of the present invention can identify a fusion protein or an object protein binding to or invading into a particular cell or protein, or a bacterium presenting the fusion protein on its surface. In this case, an extremely selective fusion protein, object protein or bacterium against target cell or protein can be identified by repeating screening using the invented library. Thus, the resulting fusion protein, object protein as a part of the fusion protein, or a bacterium presenting the fusion protein on its surface related to the present invention can specifically bind to or invade into the target cell or protein. Moreover, if the fusion protein or the object protein as a part of the fusion protein of the present invention has the ability of binding to or invading into cells, they can specifically bind to or invade into target cells.

To specifically bind to or invade into target cells as described above, the bacteria per se are not necessary, but a combination at least of the protein derived from a cell invasive protein and the object protein, or only the object protein is necessary. The fusion protein, the object protein or the bacterium of the present invention screened according to the above described procedure is extremely useful because they can specifically bind to particular and selected cells or proteins. The fusion protein, the object protein or the bacterium with cell invasiveness disclosed in the present invention is extremely useful in the property of specifically invading particular and selected cells.

Thus, by applying the fusion protein or the object protein to cells with a particular disease, it becomes possible to develop a therapeutic agent for the disease with few adverse reactions, develop a therapy for the disease, and diagnose the disease.

For example, if target cell are cancer cells, the fusion protein, the object protein or the bacterium of the present invention is added with additional biological activity, such as one which kills target cells or reverses target cells into normal cells so as to treat only cells with the disease. This enables to establish secure and safe therapeutic agent or therapy with high efficiency, and few adverse reaction against normal cells. The fusion protein, the object protein or the bacterium of the invention may have the above biological activity by itself, or may be added with a particular substance such as a agent having anti-cancer activity. For example, a drug delivery system in which a therapeutic agent can be specifically delivered against the targeted disease cells can be established by, for example, combining an agent killing cancer cells with the fusion protein, the object protein or the bacterium of the invention and by administrating it to treat only cancer cells. There is no restriction on the cell to be targeted by the above drug delivery system as long as the protein on the surface of the cell is recognized by the fusion protein, the object protein or the bacterium mainly based on the interaction between them.

Also, if target cells are cells related to autoimmune disease, an immunosuppressive agent can be combined with the fusion protein, preferably the object protein or the bacterium of the invention, which specifically binds or invades the target cells so as to prepare a therapeutic agent and therapy for autoimmune disease.

The fusion protein, the object protein or the bacterium which invades the target cell can be prepared by using the bacterial peptide library of the present invention. It is possible to establish a drug delivery system to introduce a particular substance into the targer cell by using the fusion protein, the object protein or the bacterium.

If the fusion protein or the object protein of the present with ability of invading the target cells is used, where a certain therapeutic substance may have a low therapeutic effect on the diseased cells only when existing near the surfaces of the diseased cells, but may work effectively when present within the cells, the therapeutic substance is added to the fusion protein, whereby it is taken up within the diseased cells, showing a therapeutic effect on these cells.

In the present invention, the therapeutic substance is not restricted. Depending on the kinds of diseases, various pharmaceuticals, cytotoxic substances, or compositions containing the therapeutic substances are usable. For example, protein-based substances such as physiologically active proteinaceous substances, cytokines or growth factors, or DNA such as an antisense for suppressing symptoms of disease can be used. Furthermore, there can be used immunosuppressive agents including immunosuppressants and agents for treatment of immune diseases or for use in organ transplantation, and receptors reactive tumor-specifically with cancer cells, as those of breast cancer or ovarian cancer, for which epidermal growth factor (EGF) plays an important role.

As already explained, the above diseased cells are not restricted. If cell recognition or cell invasion differs between the normal form and the diseased form of certain cells, the use of a amino acid sequence contributing the binding specific for the diseased cells (a binding signal) or a amino acid sequence contributing the invading specific for the diseased cells (an invasion signal) permits the fusion protein or a part thereof to specifically bind to or invade into the diseased cells. In the case of carcinoma (such as malignant tumor, lung cancer, leukemia, colon cancer, ovarian cancer, breast cancer or pancreatic cancer), for example, the use of the library related to the present invention disclosed above permits the identification of the object protein binding or invading only the cancerous cells. Thus, the present invention provides a system permitting the fusion protein binding to or invading into the cancer cells, and the fusion protein itself used for the system. Moreover, the present invention is applicable to autoimmune diseases (rheumatism, multiple sclerosis, systemic lupus erythematosus), allergy, AIDS, hepatitis (types B and C), and also to organ transplant cells.

Means of adding the desired therapeutic substance for diseased cells to the fusion protein are not restricted. An example is to add DNA, encoding a particular therapeutic substance for diseased cells or a derivative peptide or protein having its function, to DNA encoding the fusion protein, and suitably cut and separate the fusion protein combined with the therapeutic substance from the bacteria expressing the fusion protein (if desired, after replication of the bacteria). A preferred embodiment is to separate the fusion protein from the bacteria (if desired, after replication of the bacteria) after screening, and add a necessary therapeutic substance such as chemical compound, peptide or protein having desired physiological activity to the resulting fusion protein by any of various binding methods, thereby preparing a therapeutic agent. For this purpose, means for use with various conventional drug delivery systems can be selected suitably.

Using the resulting therapeutic agent enables disease to be treated at the cellular level and with a reduced possibility for adverse reactions.

Labeling the fusion protein, the object protein or the bacterium and making them bind to particular diseased cells enable the identification of the diseased cells by the labeling.

Furthermore, the screening method (library-based, or fusion protein or object protein-based) of the present invention based on specific binding to specific substances (proteins, etc.) produced from various diseases discloses the development of a simple and highly specific diagnostic method for the diseases. Actually, as a diagnostic method, various known assay methods can be selected, such as immunoassay in which a protein or antibody specifically produced by disease can be quantitatively determined by the screening method. Using the fusion protein, the object protein or the bacterium, which can bind particular diseased cells or substances specifically produced from disease, of the present invention enables to provide a diagnostic agent and a diagnostic kit for the disease.

The present invention will be described in detail based on the following working examples. However, these examples are offered by way of illustration, and do not limit the invention.

### EXAMPLES

### Example 1

### Construction of bacterial peptide library (E. coli surface random peptide presented library ;ESPEL)

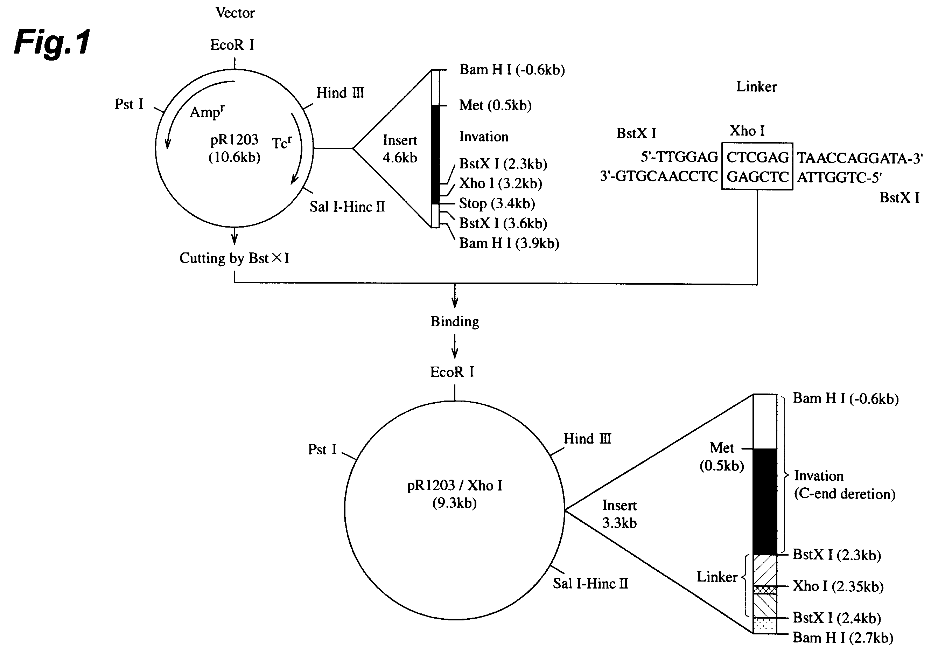

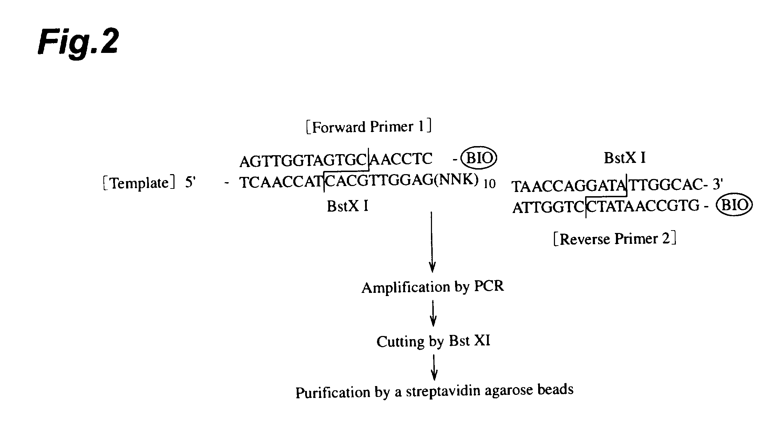

ESPEL was constructed by the methods shown in FIG. 1 and FIG. 2. That is, a plasmid vector containing DNA encoding invasin was cut at BstX I sites. A synthesized BstX I linker containing an XhoI site was introduced into the cleaved region.

DNA encoding random peptide of the object protein is synthesized in accordance with the following steps.

DNA comprising a BstX I site joined to (NNK)₁₀ was synthesized to be a template. The template was amplified by PCR with a biotinylated primer capable of annealing with the template. After digesting the PCR products with BstX I, the biotinylated primer was removed by use of strepto-avidin agarose beads.

The resulting DNA encoding the object protein was ligated to the pRI203/XhoI vector cut with BstX I. The ligation product was introduced into E. coli to transform it. Details will be offered below.

### (1)Construction of vector

### (1-1) Cutting of invasin plasmid vector with restriction enzyme

Plasmid pRI203 vector containing DNA encoding invasin, in which vector the DNA of 3.9 kb encoding invasin was inserted into BamH I site of the pBR325 vector as a platform (Cell, vol. 50, 769-778, August 28, 1987) was cut with BstX I. The cutting reaction was performed by reacting plasmid pRI203 vector with BstX I restriction enzyme for 90 minutes at 45°C. The reaction mixture after the reaction was extracted with phenol/chloroform and precipitated with isopropanol. The resulting pellet was dissolved with 1 ml of TE buffer (10mM Tris-HCl, 2mM EDTA, pH 8.0). The solution was added with 100 µl 3M sodium acetate (pH 5.6) and 2 ml cold ethanol for ethanol precipitation. The mixture was allowed to stand for 30 minutes at -80°C.

Then, the mixture was centrifuged for 10 minutes at 15,000 rpm, whereafter the resulting pellets were washed with 70% ethanol, and dissolved in TE buffer solution.

### (1-2)Preparation of BstX I linker having Xho I site

A BstX I linker having BstX I sites at both ends and an Xho I site at the center was prepared.
5'-TTGGAGCTCGAGTAACCAGGATA-3' and
5'-CTGGTTACATCGAGCTCCAACGT-3' were used as BstX I linkers. The BstX I linkers were mixed each in an amount of 10 µl at a concentration of 0.1 µg/ml, heated for 5 minutes at 95°C, and cooled for annealing.

### (1-3)Ligation

The BstX I-cut plasmid obtained in (1-1), and the annealed BstX I linker obtained in (1-2) were reacted with a DNA ligase for 12 hours at 15°C to be joined together. The treated solution was reacted with E. coli DH5α, cells rendered electrocompetent, by electroporation to transform the cells. The electroporation was performed in 0.2 cm cuvettes by means of a gene pulser (a product of BIO-RAD) under the conditions 2500 V, 40 µF, 200 ohms. The transformants were plated on LB plates each containing 50 µg/ml ampicillin (LB/Amp). From the resulting clones, clones(pRI203/Xho I) having the BstX I linker inserted therein were selected by cutting with the restriction enzymes Xho I and Pst I. On an agarose electrophoresis, pRI203/Xho I clone indicated 5.7 kb and 3.7 kb bands, and pRI203 clone indicated 6.5 kb band.

### (1-4)Cutting of vector with BstX I

The pRI203 vector (pRI203/Xho I) containing BstX I linker having XhoI site obtained in (1-3) was cut with BstX I upon reaction lasting for 90 minutes at 45°C. The reaction mixture after the reaction was increased to 100 µl with ultrapure water (Milli Q Water), and extracted with phenol/chloroform. After isopropanol precipitation and centrifugation of the extract, the resulting pellets were dissolved in the TE buffer. The solution was precipitated with ethanol, whereby the vector-DNA was purified.

### (2)Preparation of random nucleotide insert

### (2-1)Preparation of template and primers

As a template for a random nucleotide containing a BstX I site,
5'-TCAACCATCACGTTGGAG(NNK)₁₀TAACCAGGATATTGGCAC-3'
(template) was synthesized by DNA synthesizer 380B (Applied Biosystem).

Then, complementary oligonucleotide biotinylated primers corresponding to the template were synthesized. As a forward primer, biotinylated
5'-TCAACCATCACGTTGGAG-3' was selected (forward primer 1). As a reverse primer, biotinylated
5'-GTGCCAATATCCTGGTTA-3' (reverse primer 2) was similarly synthesized by the synthesizer.

The resulting oligonucleotides were purified with an oligonucleotide cartridge (Perkin-Elmer).

### (2-2)PCR reaction

The template, forward primer 1 and reverse primer 2 obtained in (2-1) were used to perform PCR reaction.

The PCR reaction was carried out over 10 cycles, each cycle comprising thermal denaturation for 4 minutes at 94°C, thermal denaturation for 1 minute 30 seconds at 94°C, annealing for 2 minutes at 50°C, and elongation for 2 minutes at 72°C.

The PCR reaction mixture was extracted with phenol/chloroform, precipitated with ethanol, and suspended in TE buffer solution (pH 8.0). Whether the reaction product emerged or not was checked by 13% polyacrylamide gel electrophoresis.

### (2-3)Enzymatic cutting

The PCT product obtained in (2-2) was reacted with BstX I restriction enzyme for 2 hours at 45°C to cut the opposite ends of DNA.

### (2-4)Purification with streptavidin agarose beads

To the enzymatically cut sample obtained in (2-3), an equal amount of streptavidin-agarose beads (a product of BRL) was added. The streptavidin-agarose beads had been washed 10 times with 0.1M NaCl/TE buffer solution to be made into a 50% suspension. The sample and the beads were reacted for 1 hour at room temperature. After centrifugation, the supernatant was recovered. The precipitated beads were washed twice with Milli Q Water, and the supernatant was recovered. This supernatant was combined with the previously recovered supernatant. The combination was extracted with phenol once, and with chloroform twice, to remove the biotinylated DNA fragment remaining in the combined supernatant. Then, the aqueous phase was concentrated with a vacuum centrifugal concentrator (SPEED VAC CONCENTRATOR) to obtain an oligonucleotide (random nucleotide insert) with the opposite ends cut at the BstX I site for encoding the object protein.

### (3)Preparation of transformant

### (3-1)Ligation

The BstX I-cut vector obtained in (1-4) and the random nucleotide insert obtained in (2-4) were joined together by reaction with a DNA ligase lasting for 12 hours at 15°C. The reaction mixture after the reaction was extracted with phenol/chloroform. The extract was precipitated with ethanol, and washed with 75% ethanol. After centrifugation, the supernatant was removed, and the residue was dissolved in Milli Q Water.

### (3-2)Electrotransformation

The reaction mixture after ligation that was obtained in (3-1) was introduced into electrocompetent cells DH5α by electroporation. The electroporation was performed in 0.2 cm cuvettes by means of a gene pulser (a product of BIO-RAD) under the conditions 2500V 40 µF, 200 ohms. After pulsing, 1 ml SOC solution was immediately added. The preparation of ESPEL was completed by this procedure. After cultivation for 1 hour, the culture was plated on LB plates each containing 50 µg/ml ampicillin (LB/Amp). After 12 hours of cultivation, the number of colonies was counted. The titer of the instantly claimed library was 1.2x10⁶. The E. coli DH5α transformant library was named ESPEL/DH-Lib and deposited with National Institute of Bioscience and Human-Technology, Agency of Industrial Science and Technology, (1-3, higashi 1-chome, Tsukuba, Ibaragi, Japan), under the accession No. FERM P-15074 on August 1, 1995. Then, on July 16 1996, it was transferred to international deposition (accession No. FERM BP-5584).

### Example 2

### Cell invasion experiments with ESPEL

To identify a signal for invasion into human cells, the following cell invasion experiments with ESPEL was conducted.

### (1) Preparation of cells

VA13 (human fibroblasts transformed with SV40, ATCC CCL-75.1) were cultured in a DME culture medium containing 10% bovine fetal serum in a 10 cm petri dish until the cells became confluent. Then, the cells were washed three times with phosphate buffer solution (PBS).

### (2) Cell invasion experiments

ESPEL prepared in Example 1 was added into 10 ml of a DME culture medium (DME (-)) free from serum and an antibiotic. Then, the culture medium was added to the cell-cultured petri dish used in (1). The mixture was cultured in an CO₂-incubator at 37°C for 3 hours to make E. coli invade the cells. The culture was washed 4 times with PBS to remove cell-noninvading clones. The washed clones were cultured for 1 hour in a DME (-) culture medium to cause complete cell invasion of clones which adhered to the cell surfaces, but did not invade the cells. Then, the cells were washed with PBS 8 times.

### (3) Eradication of cell-noninvading clones

Gentamicin (50 µg/ml) was added to a DME (-) culture medium, whereafter this culture medium was added to the petri dish prepared in (2). The mixture was incubated for 2 hours to eradicate the cells not invaded by the E. coli. Then, the cells invaded by the E. coli were washed with PBS 10 times.

### (4) Extraction of E. coli invading cells

To the petri dish prepared in (3), 2 ml of a 1% Triton-X100 aqueous solution was added, and the cells were lysed at 37°C for 10 minutes. The lysate was washed with the addition of 10 ml PBS. Then, centrifugation was performed for 5 minutes at 6,000 rpm to recover the E. coli. To the resulting pellets of the E. coli, 5 ml of an LB culture medium containing ampicillin (LB/Amp) was added, and culture was performed for 12 hours.

### (5) Extraction of plasmids

The plasmids were extracted using a commercially available plasmid extraction kit (QIAGEN).

### (6) Electroporation

To a culture medium containing electrocompetent cells DH5α in a concentration of 10⁴ cells/ml, 10 ng of the extracted plasmids was added. The mixture was subjected to electroporation that was performed in 0.2 cm cuvettes by means of BIO-RAD's gene pulser under the conditions 2500 V, 40 µF, 200 ohms. After pulsing, 1 ml SOC solution was immediately added. After cultivation for 1 hour, 9 ml LB/Amp was added, followed by culturing the mixture for 12 hours.

### (7) Cell reinvasion experiments

VA13 cell was invaded, similar to (2), by 500 µl of E. coli obtained in (6). Cell invasive E. coli were obtained in accordance with the methods described in (3) to (4).

### (8) Plasmid extraction and DNA sequencing

E. coli obtained in (7) were planted on the LB/Amp plates, and 22 of clones were randomly selected. Plasmids were extracted in the same manner as in (5). The extract was subjected to Taq cycle sequencing by means of Perkin-Elmer's DNA sequencer 373A using the primer 5'-AAGTGTTGATACCCACTTTGT-3' adjacent to the site where the object protein existed (from 1761 position to 1781 position in the Seq. ID No. 3 as in the Sequence Listing).

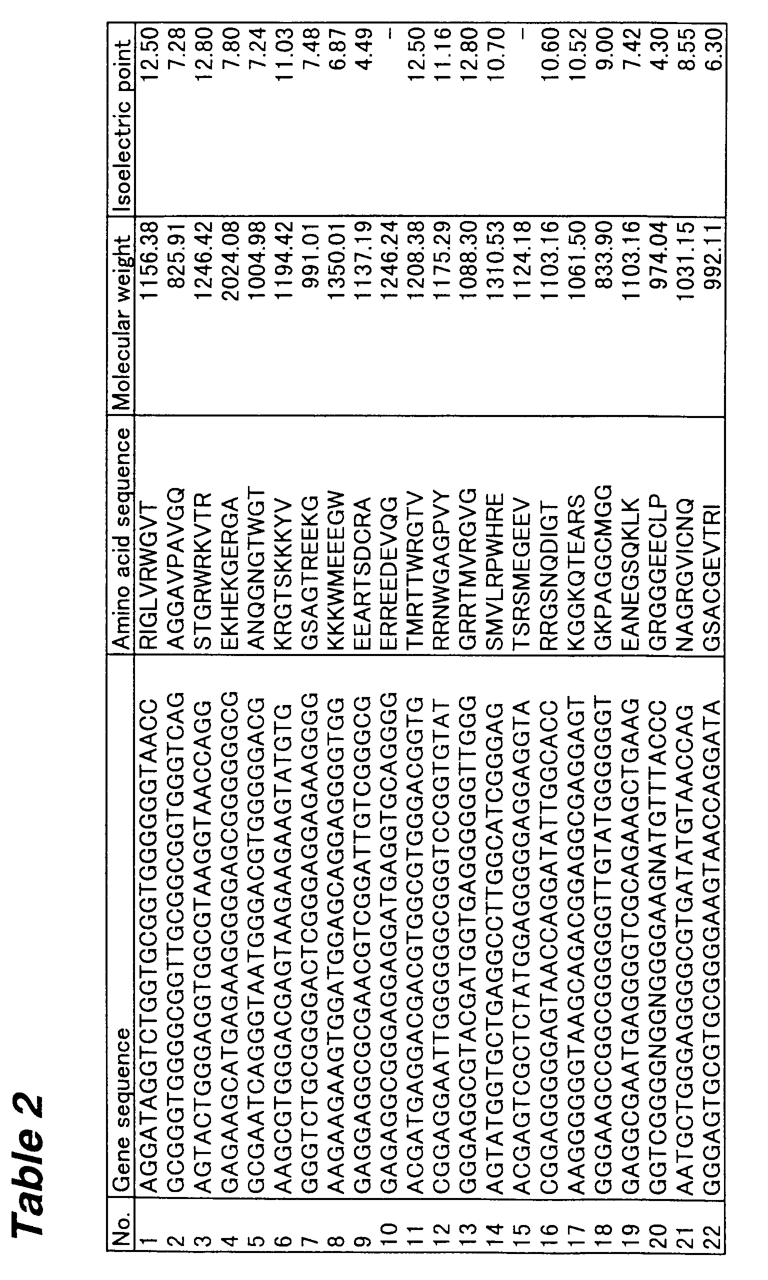

Table 2 shows the resulting DNA sequences, amino acid sequences, and the calculated values of the molecular weights and the isoelectric points of the peptides. Of the E. coli DH5α transformant library clones, the clone of No. 1 in Table 2 (base sequence and amino acid sequence: sequences of clone No. 1 in Table 2) was named ESPEL/DH-1, and deposited on August 1, 1995 with National Institute of Bioscience and Human-Technology, Agency of Industrial Science and Technology (1-3, Higashi 1-chome, Tsukuba, Ibaragi, Japan), under the accession No. FERM P-15073. Then, on July 16, 1996, it was transferred to international deposition (accession No. FERM BP-5583).

### Example 3

### Construction of cysteine looped E. coli surface random peptide presented library (CL-ESPEL)

To present the object protein efficiently, a spacer protein comprising six prolines was joined to the support protein. Further, cysteine residues were added to the opposite ends of the random peptide portion, thereby to construct CL-ESPEL forming the loop via the SH groups of the cysteines.

The construction of the cysteine looped ESPEL was performed in the same manner as in Example 1, except the preparation of the oligonucleotide insert.

That is, an invasin plasmid vector incorporating a BstX I linker having XhoI site (pRI203/Xho I) was prepared as in Example 1.

Separately, random oligonucleotide insert was prepared by the following steps. DNA encoding a random peptide portion including BstX I site and spacer proteins consisting of six prolines and cysteine at each end of the random peptide was synthesized as a template. This DNA was amplified by PCR using a biotinylated primer annealing to the template. The PCR product was cut with BstX I, and purified through the step that biotinylated primer bound streptoavidin agarose beads.

The resulting random oligonucleotide insert was ligated to the vector cut with BstX I. The ligation product was introduced into E. coli by electroporation to transform it. Details will be offered below.

### (1) Preparation of vector

As in Example 1(1), an invasin vector having a BstX I linker inserted therein was cut with BstX I, and purified.

### (2) Preparation of random oligonucleotide insert

### (2-1)Preparation of template and primers

As a template for a random nucleotide containing a BstX I site, 
5'-

TCAACCATCACGTTGGAGCCGCCTCCACCTCCACCGGAGATCTTGGCATGC(NNK
 
)₁₀TGCTGACTGACTGAGCGGCCGCCAGGATATTGGCACC-3' (encoding
STITLEPPPPPPEILACXXXXXXXXXXC* as a protein, *: a stop
codon, AGATCT:Bgl II site, GCGGCCGC: Not I site)
 was synthesized by DNA synthesizer 380B (Perkin Elmer).

Since the object protein portion of the library has Bgl II (compatible to BamH I) site and Not I site inserted therein, the recombination of the portion into the Bgl II (or BamH I)/Not I site of another vector can be made. To amplify the peptide portion as the object protein in a large amount, the recombination of it into a high expression vector should be recommended.

For example, as a high expression vector, GST fusion vector pGEX-5X-1 (containing Factor Xa site, Pharmacia) is useful, and the random peptide portion can be recombined into the BamH I/Not I site of the vector.

That is, a plasmid is prepared from a E. coli clone obtained by the screening, and the resulting plasmid is cut at the sites of Bgl I and Not I. The resulting cut plasmid is recombined into the pGEX-5X-1, the peptide portion is expressed as a GST fusion protein in a large amount. After purifying the resulting GST fusion protein, the protein is cut with Factor Xa. Then the resulting product is subject to ultrafiltration so as to prepare the peptide portion in a large amount.

Then, complementary oligonucleotide bionylated primers corresponding to the templates were synthesized. As a forward primer, biotinylated 5'-CTCCAACGTGATGGTTGA-3' (forward primer 1) was selected. As a reverse primer, biotinylated 5'-GGTGCCAATATCCTGGCG-3' (reverse primer 2) was similarly synthesized and purified as in Example 1.

The template, forward primer 1 and reverse primer 2 obtained were used to perform PCR reaction, and the reaction product was purified with streptavidin agarose beads, as in Example 1.

### (2-2) Transformation of E. coli

Random DNA obtained in (2-1) was cut with BstX I, and purified in the same way as in Example 1. The purified DNA fragment was ligated to the BstX 1-cleaved pRI203/Xho I vector. Then, the ligation product was introduced into E. coli SURE 2 (Clonetech) by electrotransformation to produce transformants.

### (3) Gene sequencing of E. coli clone

The transformed E. coli cells were seeded on the LB/Amp plates, and randomly sampled 48 clones (480 amino acids) were subjected to Taq cycle sequencing using the primer 5'-TGCGGTACCGACATCTATCAT-3' adjacent to the site where the object protein existed (from 1818 position to 1838 position in the Seq. ID No. 3 as in the Sequence Listing),as in Example 2.

It was found that random amino acids were expressed which virtually coincided with the theoretical values expected from the randomness of DNA as shown in Table 3.

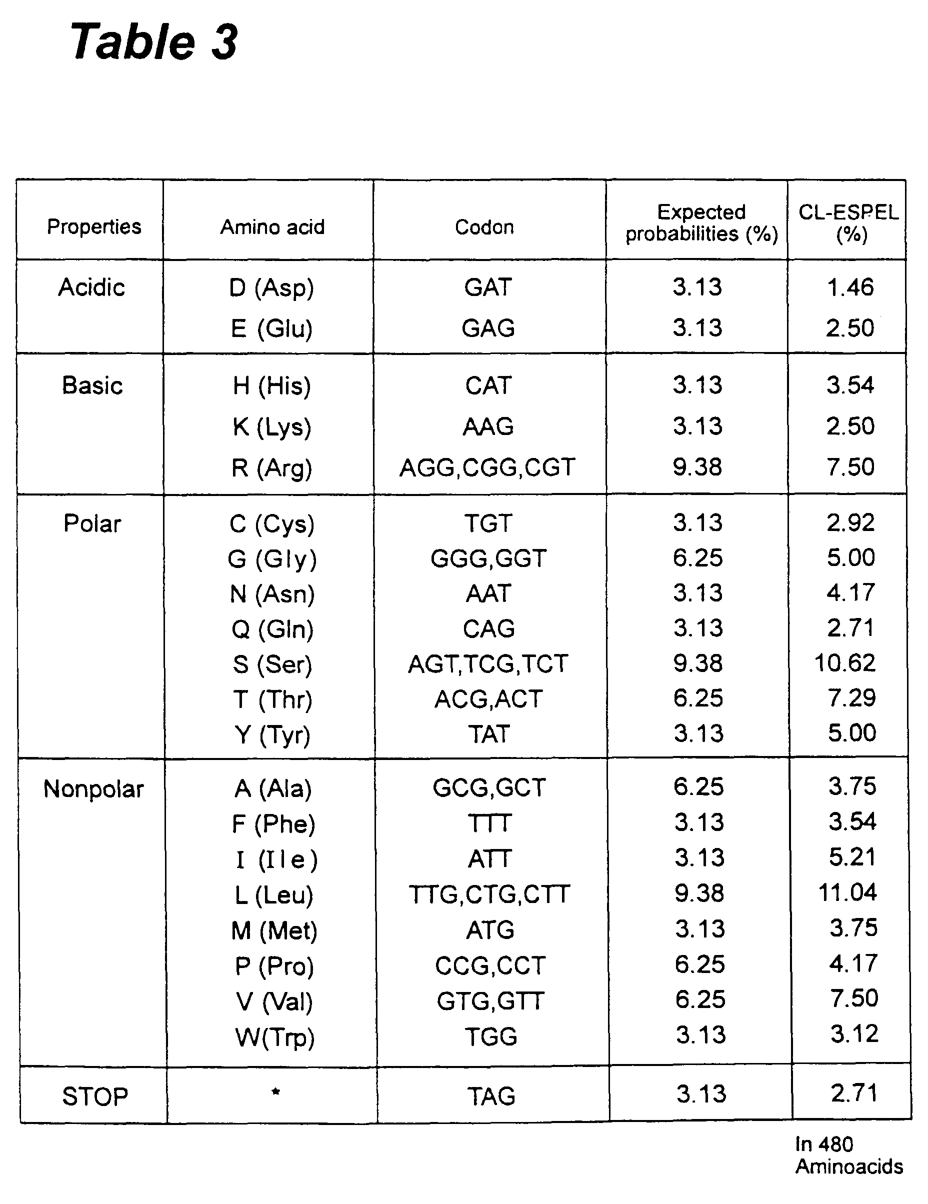

The E. coli SURE 2 transformant library was named ESPEL/SURE-Lib, and internationally deposited on July 16, 1996 with National Institute of Bioscience and Human-Technology, Agency of Industrial Science and Technology (1-3, Higashi 1-Chome, Tsukuba, Ibaragi, Japan), under the accession No. FERM BP-5582.

### Example 4

### Construction of control clone for cysteine looped E. coli surface random peptide presented library (CL-ESPEL)

As control clones for CL-ESPEL, SURE2 E. coli clones expressing (1) RGD peptide (RGDGNRGDAW) with cell binding ability, or (2) the epitope sequence (LYPYDVPDYA) recognized by anti-hemeargutinine (HA) monoclonal antibody (Mol. Cell. Biol., 8, 2159-2165, 1988), respectively, as the object protein portion, were constructed.

The construction of control clones was performed in the same manner as in Example 3, except the preparation of the oligonucleotide insert.

That is, an invasin plasmid vector incorporating a BstX I linker having XhoI site (pRI203/XhoI) was prepared as in Example 3.

Separately, random oligonucleotide insert was prepared by the following steps. DNA encoding a object peptide portion (here, RGD peptide or HA epitope) including BstX I site and spacer proteins consisting of six prolines and cysteine at each end of the random peptide was synthesized and was annealed.

The resulting random oligonycleotide insert was ligated to the vector cut with BstX I. The ligation product was introduced into E. coli by electroporation to transform it. Details will be offered below.

### (1) Preparation of vector

As in Example 1(1), an invasin vector (pRI203/Xho I) having a BstX I linker inserted therein was cut with BstX I, and purified.

### (2) Construction of control clones

### (2-1)Preparation of linker

As oligonucleotide including BstX I site, the following linkers were synthesized.
(A) linkers for RGD peptide (RGDGNRGDAW) with cell binding ability 
RGD linker 1

5'-TTGGAGCCGCCTCCACCTCCACCGGAGATCTTGGCATGCCGCGGTGACGGTT

GGCGTGGCGATGCTTGGTGCTGACTGACTGAGCGGCCGCCAGGATA -3'

RGD linker 2

5'-CTGGCGGCCGCTCAGTCAGTCAGCACCAAGCATCGCCACGCCAACCGTCACC

GCGGCATGCCAAGATCTCCGGTGGAGGTGGAGGCGGCTCCAACGTG -3'

(encoding STITLEPPPPPPEILAC<RGDGNRGDAW>C* as a

protein, *:stop codon)
(B) linkers for the epitope sequence (LYPYDVPDYA) recognized by anti-hemeagrutinin (HA) monoclonal antibody

HA linker 1

5'-TTGGAGCCGCCTCCACCTCCACCGGAGATCTTGGCATGCCTGTATCCTTATG

ACGTGCCTGACTATGCCTGCTGACTGACTGAGCGGCCGCCAGGATA -3'

HA linker 2

5'-CTGGCGGCCGCTCAGTCAGTCAGCAGGCATAGTCAGGCACGTCATAAGGATA

CAGGCATGCCAAGATCTCCGGTGGAGGTGGAGGCGGCTCCAACGTG -3'

(encoding STITLEPPPPPPEILAC<LYPYDVPDYA>C* as a protein,

*:stop codon).

Each DNA was synthesized by use of DNA synthesizer 380B (Perkin Elmer) and purified with SDS-polyacrylamide electrophoresis.

RGD linker 1 and 2 were annealed together by mixing each 10 µl of each solution at the concentration of 0.1 µg/ml, heating at 95°C for 5 minutes and cooling it.

HA linker 1 and 2 were annealed together in the same way as for RGD linkers.

### (2-2) Transformation of E. coli

Linker DNA obtained in (2-1) was ligated to the Bst X 1-cleaved pRI203/Xho I vector obtained in (1).

Then, the ligation product was introduced into E. coli SURE 2 (Clonetech) by electrotransformation to produce transformants.

### (3) Gene sequencing of E. coli clone

The transformed E. coli cells were seeded on the LB/Amp plates, and randomly sampled several clones were subjected to Taq cycle sequencing using the primer 5'-TGCGGTACCGACATCTATCAT-3' adjacent to the site where the object protein existed (from 1818 position to 1838 position in the Seq. ID No. 3 as in the Sequence Listing),as in Example 2.

It was found that the RGD clone and the HA clone, having each object linker introduced therein, respectively, were obtained. They were used as control clone for the following experiments.

### Example 5

Cell binding experiment with cystein looped E. coli surface random peptide presented library (CL-ESPEL)

To identify a signal for binding to human cells, the following cell invasion experiment with CL-ESPEL was conducted.

### (1) Preparation of cells

VA13 cells were cultured in a DME culture medium containing 10% bovine fetal serum in a 6-well multi-well plate until the cells became confluent (0.5 x 10⁷ cells/well). Then, the plate was washed three times with DME culture medium free from serum and an antibiotics (DME(-)).

### (2) Cell binding experiments

CL-ESPEL prepared in Example 3 was suspended into DME(-) culture medium containing 3% of bovine serum albumin (DME/BSA) to be a concentration of (5 x 10⁷ cells/ml). Then, the resulting suspension was added to each well of the plate used in (1). The mixture was cultured in an CO₂-incubator at 37°C for 2 hours to make E. coli bind to (and also invade into) the cells. The plate was washed 10 times with DME(-) to remove cell-nonbinding clones.

### (3) Extraction of E. coli binding cells

To each well of the plate prepared in (2), 1 ml of a 1% Triton-X100 aqueous solution was added, and the cells were lysed at 37°C for 10 minutes. Then, centrifugation was performed for 5 minutes at 6,000 rpm to recover the E. coli, and the resulting pellet was washed once with DME(-). To the resulting pellets of the E. coli, 1 ml of an LB culture medium was added, and culture lasted 10 minutes. Then a part of the resulting cultures medium was planted on the LB agarose plate containing ampicillin (LB/Amp), and culture was performed for 12 hours, and then colonies appeared were counted. The remaining part of the suspension was cultured in LB culture medium containing ampicillin for 12 hours, and the cultured E. coli was suspended in DME/BSA at a concentration of 5 x 10⁷ cells/ml.

### (4) Cell rebinding experiments

VA13 cell was bound, similar to (2), with E. coli obtained in (3), and cell-binding clones were obtained. Moreover, the binding experiment with VA13 cell was repeated. As a total, 3 times of the binding experiment were completed.

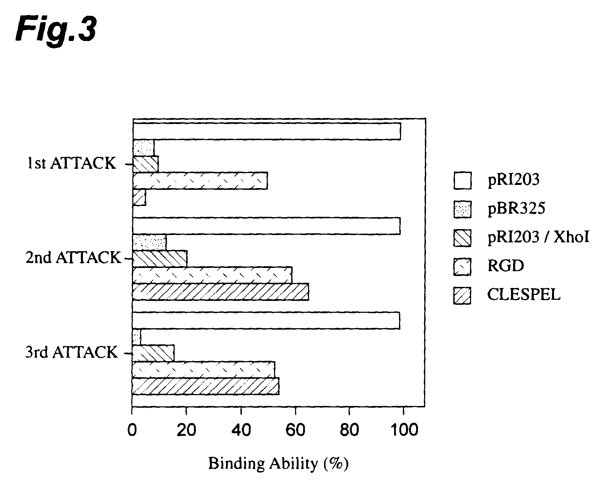

Fig. 3 shows the process of selection of Cl-ESPEL with stronger binding ability depending on the repeat of the binding experiment in compared with the result of control clones.

In the above experiment, the invasin expression clone (the SURE2 clone having pRI203 introduced therein disclosed in Example 1) and RGD clone obtained in Example 4 were used as positive controls. The clone having only the original vector (the SURE2 clone having the original pBR325 vector introduced therein, which vector was used for the gene recombination experiment in Example 1) and the clone having only the support protein (the SURE2 clone having pRI203/XhoI vector introduced therein in Example 1) were used as negative controls.

When the binding ability of the invasin expression clone as a positive control was standardized as 100 %, the binding ability of RGD clone is 60% and this value was not varied during the three times of the binding experiments. The clone with only the vector and the clone with only the support protein as negative controls shows their binding ability less than 20 % and those values were not varied during the repeat of the binding experiments.

In the binding experiment using CL-ESPEL, while the result of the first experiment showed 10 % of binding ability, the results of the second and third experiments showed more than 50 %. This means that a cluster of E. coli with cell binding ability was specifically selected (Fig. 3).

### (5) Plasmid extraction and DNA sequencing

CL-ESPEL obtained after the three times of binding experiments in (4) were planted on the LB/Amp plates, and 15 of clones were randomly selected. The selected clones were cultured in liquid culture medium, respectively. Plasmids were extracted and purified from the resultant cultured E coli. with a commercially available plasmid extraction kit (QIAGEN). The purified plsmid was subjected to Taq cycle sequencing using the primer 5'-TGCGGTACCGACATCTATCAT-3' adjacent to the site where the object protein existed (from 1818 position to 1838 position in the Seq. ID No. 3 as in the Sequence Listing).

### (6) Evaluation of binding ability of each clone

The binding ability of each clone obtained to VA13 cell was evaluated by the following steps: Each clone was subject to the cell binding experiment as the same way as in (1) and (2). The binding clone was selected by the same way as in (3). The resulting clone was planted on LB/Amp agarose plate and cultured for 12 hours. The appeared colonies were counted to calculate their binding ability.

The resulting DNA sequences and amino acid sequences are shown in Table 4.

As a result, three amino acid sequences of clones binding VA13 cell, K1:GPSVITSCSI (62.0% of pRI203), K2:SNFLTQRXSM (50.6% of pRI203) and K21:GQSVITSGSI (33.1% of pRI203) were obtained. K1 clone had stronger binding ability than RGD clone (53.5% of pRI203). The above results indicates that E. coli clone with strongly cell binding ability can be obtained by use of the screening system of the present invention.

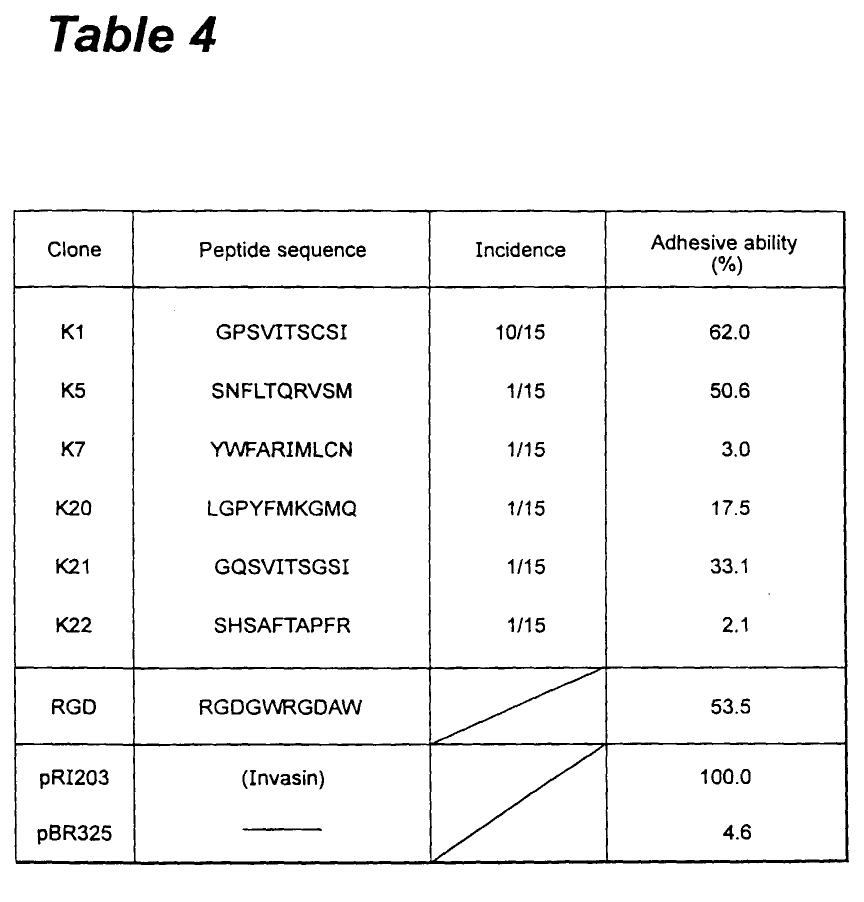

### Example 6

### Cell invasion experiments with cystein looped E. coli surface random peptide presented library (CL-ESPEL)

To identify a signal for invasion into human cells, the following cell invasion experiments with CL-ESPEL was conducted.

### (1) Preparation of cells

VA13 cells were cultured in a DME culture medium containing 10% bovine fetal serum in a 6-well multi-well plate until the cells became confluent (0.5 x 10⁷ cells/well). Then, the plate was washed three times with DME culture medium free from serum and an antibiotics (DME(-)).

### (2) Cell invasion experiments

CL-ESPEL prepared in Example 3 was suspended into DME(-) culture medium containing 3% of bovine serum albumin (DME/BSA) to be a concentration of (5 x 10⁷ cells/ml). Then, the resulting suspension was added to each well of the plate used in (1). The mixture was cultured in an CO₂-incubator at 37°C for 2 hours to make E. coli invade into the cells. The plate was washed 10 times with DME(-) to remove cell-noninvading clones.

### (3) Eradication of cell-noninvading clones

Gentamicin (50 µg/ml), Penicillin (100 U/ml) and Streptomycine (100 µg/ml) was added to a DME (-) culture medium, whereafter this culture medium was added to each well of the plate prepared in (2). The mixture was incubated for 2 hours to eradicate E. coli not invading VA13 cell. Then, the plate was washed with PBS 10 times.

### (4) Extraction of E. coli invading cells

To each of the plate prepared in (2), 1 ml of a 1% Triton-X100 aqueous solution was added, and the cells were lysed at 37°C for 10 minutes. The lysate was centrifuged for 5 minutes at 6,000 rpm to recover the invading E. coli. To the resulting pellets of the E. coli, 1 ml of a LB culture medium was added, and culture was performed for 10 minutes. A part of the cultured medium was planted on a LB/Amp agarose plate and culture was performed for 12 hours to count colonies appeared. The remaining part of the cultured medium was cultured in liquid LB culture medium containing ampicillin, and the cultured E. coli was suspended in DME/BSA at a concentration of 5 x 10⁷ cells/ml.

### (5) Cell reinvading experiments

VA13 cell was invaded, similar to (2), with E. coli obtained in (4), and cell-invading clones were obtained. Moreover, the invading experiment with VA13 cell was repeated. As a total, 3 times of the invading experiment were completed.

### (6) Plasmid extraction and DNA sequencing

CL-ESPEL obtained after the three times of binding experiments in (5) were planted on the LB/Amp plates, and 7 of clones were randomly selected. The selected clones were cultured in liquid culture medium, respectively. Plasmids were extracted and purified from the resultant cultured E coli. with a commercially available plasmid extraction kit (QIAGEN). The purified plsmid was subjected to Taq cycle sequencing using the primer 5'-TGCGGTACCGACATCTATCAT-3' adjacent to the site where the object protein existed (from 1818 position to 1838 position in the Seq. ID No. 3 as in the Sequence Listing).

As a result, clones invading into VA13, SRSWPVYVLS (9 clones of the 10 clones) and ILSIPYVNQA (1 clone of the 10 clones), were obtained.

### Example 7

### Antibody epitope binding experiments using cystein looped E. coli surface random peptide presented library (CL-ESPEL)

It is confirmed that an epitope of an antibody presented in the object protein portion of the cysteine looped ESPEL (CL-ESPEL) can be detected an antibody recognizing the epitope.

### (1) Preparation of clones expressing an antibody epitope

The clone (HA clone), expressing the epitope sequence (LYPYDVPDYA) recognized by anti-hemeagrutinin (HA) monoclonal antibody obtained in Example 4 , was cultured in LA/Amp culture medium. The RGD clone including RGD peptide (RGDGNRGDAW) having cell binding ability, was also cultures as a control.

### (2) Detection of epitope expressed E. coli by color development reaction using an antibody

The HA clone and the RGD clone, respectively, were washed three times with PBS containing 0.5% of Tween 20 (PBS/Tween), and then were fixed with 70% ethanol for 30 minutes. After blocking with Blockace™(Yukijirushi, Japan), the fixed E.coli was reacted with anti-hemeagrutinin (HA) monoclonal antibody (10 µg/ml) for 10 minutes at room temperature. Then, after washing three times with PBS/Tween, the E.coli was reacted with biotinylated anti-mouse IgG (1 µg/ml) for 1 hour at room temperature. After washing three times with PBS/Tween, the E.coli was reacted with peroxidase conjugated streptoavidin antibody (1 µg/ml) for 30 minutes at room temperature. After washing the reacted E.coli with PBS/Tween, ABTS solution (0.3 mg/ml ABTS, 0.006% H₂O₂, 100mM citrate buffer, pH 4.0) was added as a substrate solution, whereby color was developed. The results showed that HA clone presented green color and RGD clone presented nothing. The presentation of the epitope in HA clone was confirmed by this result.

### (3) Detection of epitope expressed E.coli by filter hybridization

The HA clone, or RGD clone as a control, was planted on LA/Amp plate at a low density(50-200cells/10 cm petri dish) and cultured at 37°C for 12 hours. A nylon filter (Amersham, Hybond N+™) was layered on the plate where colonies appeared on, and allowed to stand for 30 minutes. The filter was peeled off and dried on a filter paper. After washing with PBS/Tween, the nylon filter was fixed with 70% ethanol.

After blocking with Blockace™, the fixed nylon filter was reacted with anti-HA antibody (10 µg/ml) as a primary antibody. After washing with PBS/Tween, the reacted filter was further reacted with peroxidase conjugated anti-mouse IgG antibody (1 µg/ml), as a secondary antibody for 1 hour at room temperature. The reacted filter was washed PBS/Tween. Then the pattern of the filter exposed on a film by use of ECL system (Amersham).

Almost all of the HA clones were observed as black spots as positive, while RGD clones were negative.

These results indicates that the object protein portion of the cystein looped ESPEL (CL-ESPEL) can be recognized by a protein such as antibody, and the CL-ESPEL is useful for an experiment for identifying a binding site of a protein.

## Claims

1. A bacterial surface-presented fusion protein, characterized in that at least a part of said fusion protein is presented on the surfaces of bacteria and said fusion protein comprises a protein derived from a cell invasive protein and an object protein.

2. A bacterial surface-presented fusion protein, characterized in that at least a part of said fusion protein is presented on the surfaces of bacteria and said fusion protein comprises a protein derived from a cell invasive protein and an object protein combined with the C-terminal portion of said protein derived from a cell invasive protein.

3. The bacterial surface-presented fusion protein of Claim 1 or 2, wherein the fusion protein includes at least spacer.

4. The bacterial surface-presented fusion protein of Claim 3, wherein the spacer is located between the protein derived from a cell invasive protein and the object protein.

5. The bacterial surface-presented fusion protein of Claim 3 or 4, wherein the spacer comprises 3 to 10 consecutive prolines.

6. The bacterial surface-presented fusion protein of any one of Claim 3 to 5, wherein the spacer contains an enzyme digestion site.

7. The bacterial surface-presented fusion protein of any one of Claim 1 to 6, wherein the cell invasive protein is selected from the group consisting of invasin or ail of Yersinia pseudotuberculosis, invaisin or ail Yersinia Enterocorytecarium, inv A, B, C, D or H of Salmonella typhimurium, ipa B, ipa C, ipa D or hil of Shigella flexneri, eae or cfm of pathogenic Escherichia coli, and a cell invasive protein presented on the surface of Leishmania.

8. The bacterial surface-presented fusion protein of any one of Claim 1 to 6, wherein the cell invasive protein is the protein having an amino acid sequence described as Seq. ID No. 1 in the Sequence Listing.

9. The bacterial surface-presented fusion protein of any one of Claim 1 to 6, wherein the protein derived from a cell invasive protein is the protein having an amino acid sequence described as Seq. ID No. 2 in the Sequence Listing.

10. The bacterial surface-presented fusion protein of any one of Claim 7 to 9, wherein the protein derived from a cell invasive protein is a modified protein.

11. The bacterial surface-presented fusion protein of any one of Claim 1 to 10, wherein the object protein has a ligand effect on a receptor.

12. The bacterial surface-presented fusion protein of any one of Claim 1 to 10, wherein the object protein has cell binding ability.

13. The bacterial surface-presented fusion protein of any one of Claim 1 to 12, wherein the protein derived from a cell invasive protein loses cell invasiveness which the cell invasive protein inherently has.

14. The bacterial surface-presented fusion protein of Claim 13, wherein the fusion protein has cell invasiveness.

15. The bacterial surface-presented fusion protein of any one of Claim 1 to 14, characterized in that the object protein comprises random amino acid sequence.

16. The bacterial surface-presented fusion protein of Claim 15, characterized in that the object protein comprises random amino acids sequence having 3 - 16 amino acids length.

17. The bacterial surface-presented fusion protein of any one of Claim 1 to 16, characterized in that the object protein includes at least 2 cystein residues and at least two of the cystein residues form a disulfide bond.

18. A method for presenting a fusion protein on the surface of bacteria, which comprises into vectors inserting DNA encoding said fusion protein comprising a protein derived from a cell invasive protein and an object protein, introducing said vectors into bacteria to transform the bacteria, and expressing said fusion protein.

19. A method for presenting a fusion protei on the surface of bacteria, which comprises:
into vectors inserting DNAs encoding said fusion protein comprising a protein derived from a cell invasive protein and an object protein, wherein said DNA comprising at least DNA sequence encoding the protein derived from a cell invasive protein and DNA sequence encoding the object protein located on 3' side of said DNA sequence encoding the protein derived from a cell invasive protein;
introducing said vectors into bacteria to transform the bacteria;
and expressing said fusion protein

20. The method for presenting a fusion protein on the surface of bacteria of Claim 18 or 19, characterized in that the DNA encoding the fusion protein includes DNA sequence encoding a spacer.

21. The method for presenting a fusion protein on the surface of bacteria of any one of Claim 18 to 20, characterized in that the DNA sequence encoding the protein derived from a cell invasive protein is a sequence encoding a protein derived from the protein selected from the group consisting of inv or ail of Yersinia pseudotuberculosis, invasin or ail of Yersinia Enterocorytecarium, inv A, B, C, D or H of Salmonella typhimurium, ipa B, ipa C, ipa D or hil of Shigella flexneri, eae or cfm of pathogenic Escherichia coli, and a cell invasive protein presented on the surface of Leishmania.

22. The method for presenting a fusion protein on the surface of bacteria of any one of Claim 18 to 21, characterized in that
the DNA sequence encoding the protein derived from a cell invasive protein is the DNA sequence described as Seq. ID No. 3 in the Sequence Listing.

23. The method for presenting a fusion protein on the surface of bacteria of any one of Claim 18 to 21, characterized in that the DNA sequence encoding the object protein is DNA sequence of the formula (NNK)ₙ where N is one of deoxyribonucleotides being A, G, C and T, K is one of deoxyribonucleotides being G and T, and n denotes an integer at least 3.

24. The method for presenting a fusion protein on the surface of bacteria of Claim 23, characterized in that the DNA sequence encoding the object protein is DNA sequence of the formula (NNK)ₙ where N is one of deoxyribonucleotides being A, G, C and T, K is one of deoxyribonucleotides being G and T, and n denotes an integer of 3 to 16.

25. The bacterial surface-presented fusion protein presented by the method of any one of Claim 18 to 24.

26. A method for preparing an object protein, which comprises the following steps:
(a) inserting DNA encoding a fusion protein into vectors, which fusion protein comprises a protein derived from a cell invasive protein, an object protein and a spacer, consisting of amino acids having enzyme digestion site, located between both proteins,
(b) introducing the vectors into bacteria to transform the bacteria and presenting said fusion protein on the surfaces of bacteria,
(c) multiplying the transformants,
(d) digesting the spacer of said fusion protein presented on the bacterial surface by use of the enzyme, and
(e) isolating the object protein cut off from the bacteria.

27. The method for preparing the object protein of Claim 26, characterized in that in the DNA encoding the fusion protein comprising a protein derived from a cell invasive protein, an object protein and a spacer, consisting of amino acids having an enzyme digestion site, located between both proteins, the DNA sequence encoding the protein derived from a cell invasive protein is located on the 5'-side of the DNA sequence encoding the spacer and the DNA sequence encoding the spacer is located on the 5'-side of the DNA sequence encoding the object protein.

28. The method for preparing the object protein of Claim 26 or 27, characterized by further including the following step after the step (c); (c-1) screening the transformant presenting desired fusion protein on the surfaces of bacteria.

29. The object protein produced by the method of any one of Claim 26 to 28.

30. DNA encoding a fusion protein comprising a protein derived from a cell invasive protein and an object protein.

31. DNA encoding a fusion protein comprising a protein derived from a cell invasive protein and an object protein, wherein said DNA comprising at least DNA sequence encoding the protein derived from a cell invasive protein and DNA sequence encoding the object protein located on 3' side of DNA sequence encoding the protein derived from a cell invasive protein.

32. DNA of Claim 30 or 31, characterized in that the DNA sequence encoding the protein derived from a cell invasive protein is the base sequence described as Seq. ID No.3 in the Sequence Listing and DNA encoding the object protein is DNA sequence of the formula (NNK)ₙ where N is one of deoxyribonucleotides being A, G, C and T, K is one of deoxyribonucleotides being G and T, and n denotes an integer of 3 to 16.

33. DNA of Claim 32 characterized in that n is 10.

34. DNA of any one of Claim 30 to 33, characterized in that DNA encoding a spacer is located between DNA encoding the protein derived from a cell invasive protein and DNA encoding the object protein.

35. DNA encoding the bacterial surface-presented fusion protein of any one of Claim 1 to 17.

36. A bacterium having the bacterial surface-presented fusion protein of any one of Claim 1 to 17.

37. A bacterium made by the method for presenting the fusion protein on the surface of bacterial cells of any one of Claim 18 to 24.

38. The bacterium of Claim 36 or 37, characterized in that the bacterium is gram-negative.

39. The bacterium of Claim 38, characterized in that the gram-negative bacterium is E.coli.

40. A bacterial peptide library as a collection of bacteria described in any one of Claim 36 to 39.

41. The bacterium of any one of Claim 36 to 39, characterized in that the fusion protein has ligand effect on receptor.

42. The bacterium of any one of Claim 36 to 39, characterized in that the fusion protein has cell invading ability.

43. The bacterium of any one of Claim 36 to 39, characterized in that the fusion protein has cell invasiveness.

44. The bacterium of any one of Claim 36 to 39, characterized in that the fusion protein has ligand effect on receptor and cell binding ability.

45. The bacterium of any one of Claim 36 to 39, characterized in that the fusion protein has ligand effect on receptor and cell invasiveness.

46. A method for identifying an object protein having biological activity by the bacterial peptide library of Claim 40.

47. The method of Claim 46, characterized in that the biological activity is cell binding ability.

48. The method of Claim 46, characterized in that the biological activity is cell invasiveness.

49. The method of Claim 46, characterized in that the biological activity is ligand effect on receptor.

50. The object protein having biological activity identified by the method of any one of Claim 46 to 49.

51. DNA encoding the object protein of Claim 50.

52. A method for identifying a fusion protein having biological activity by the bacterial peptide library of Claim 40.

53. The method of Claim 52, characterized in that the biological activity is cell binding ability.

54. The method of Claim 52, characterized in that the biological activity is cell invasiveness.

55. The method of Claim 52, characterized in that the biological activity is ligand effect on receptor.

56. A fusion protein having biological activity identified by the method of any one of Claim 52 to 55.

57. DNA encoding the object protein of CLaim 56.

58. A therapeutic agent for disease comprising at least a part of the object protein of Claim 29 or 50 and a therapeutic substance.

59. The therapeutic agent consisting of the object protein of Claim 29 or 50.

60. The therapeutic agent of Claim 58 or 59, wherein the disease is selected from autoimmune disease or cancer.

61. The diagnostic kit for cancer comprising at least a part of the object protein of Claim 29 or 50.

62. The diagnostic agent or diagnostic kit of Claim 61, wherein the disease is selected from autoimmune disease or cancer.

63. The therapeutic agent comprising at least a part of the fusion protein of Claim 56 and a therapeutic substance.

64. The therapeutic agent consisting of at least a part of the fusion protein of Claim 56.

65. The therapeutic agent of Claim 63 or 64, wherein the disease is selected from autoimmune disease or cancer.

66. The diagnostic agent or diagnostic kit comprising at least a part of the fusion protein of Claim 56.

67. The diagnostic agent or diagnostic kit of Claim 66, wherein the disease is selected from autoimmune disease or cancer.

68. A recombinant vector comprising DNA encoding the fusion protein of Claim 56.

69. The recombinant vector of Claim 68, characterized in that the vector is a plasmid vector.

70. A diagnostic method for diagnosing a disease by using at least a part of the object protein of Claim 29 or 50.

71. The diagnostic method of Claim 70, wherein the disease is selected from autoimmune disease or cancer.

72. A therapy for treating a disease by using at least a part of the object protein of Claim 29 or 50 and a therapeutic agent.

73. The therapy of Claim 72, wherein the disease is selected from autoimmune disease or cancer.

74. A diagnostic method for diagnosing a disease by using at least a part of the fusion protein of Claim 56.

75. The diagnostic method of Claim 74, wherein the disease is selected from autoimmune disease or cancer.

76. The therapy for treating a disease by using at least a part of the fusion protein of Claim 56.

77. The therapy of Claim 76, wherein the disease is selected from autoimmune disease or cancer.


SEQUENCE LISTING

(1) GENERAL INFORMATION:

(i) APPLICANT:
(A) NAME: SUMITOMO ELECTRIC INDUSTRIES, LTD.
(B) STREET: 5-33 Kitama 4-chrome
(C) CITY: Chuo-ku
(D) STATE: Osaka-shi
(E) COUNTRY: Osaka 541-0041 Japan
(F) POSTAL CODE (ZIP): none

(ii) TITLE OF INVENTION: BACTERIAL PEPTIDE LIBRARY

(iii) NUMBER OF SEQUENCES: 3

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 96925994.4
(vi) PRIOR APPLICATION DATA:
(A) APPLICATION NUMBER: JP 7/199745
(B) FILING DATE: 04-AUG-1995

(2) INFORMATION FOR SEQ ID NO: 1:

(i) SEQUENCE CHARACTERISTICS:
(A) LENGTH: 986 amino acids
(B) TYPE: amino acid
(C) STRANDEDNESS:
(D) TOPOLOGY: linear

(ii) MOLECULE TYPE: protein

(xi) SEQUENCE DESCRIPTION: SEQ ID NO: 1:

Met Met Val Phe Gln Pro Ile Ser Glu Phe Leu Leu Ile Arg Asn Ala
1               5                   10                  15

Gly Met Ser Met Tyr Phe Asn Lys Ile Ile Ser Phe Asn Ile Ile Ser
            20                  25                  30

Arg Ile Val Ile Cys Ile Phe Leu Ile Cys Gly Met Phe Met Ala Gly
        35                  40                  45

Ala Ser Glu Lys Tyr Asp Ala Asn Ala Pro Gln Gln Val Gln Pro Tyr

50                  55                  60

Ser Val Ser Ser Ser Ala Phe Glu Asn Leu His Pro Asn Asn Glu Met
65                  70                  75                  80

Glu Ser Ser Ile Asn Pro Phe Ser Ala Ser Asp Thr Glu Arg Asn Ala
                85                  90                  95

Ala Ile Ile Asp Arg Ala Asn Lys Glu Gln Glu Thr Glu Ala Val Asn
            100                 105                 110

Lys Met Ile Ser Thr Gly Ala Arg Leu Ala Ala Ser Gly Arg Ala Ser
        115                 120                 125

Asp Val Ala His Ser Met Val Gly Asp Ala Val Asn Gln Glu Ile Lys
    130                 135                 140

Gln Trp Leu Asn Arg Phe Gly Thr Ala Gln Val Asn Leu Asn Phe Asp
145                 150                 155                 160

Lys Asn Phe Ser Leu Lys Glu Ser Ser Leu Asp Trp Leu Ala Pro Trp
                165                 170                 175

Tyr Asp Ser Ala Ser Phe Leu Phe Phe Ser Gln Leu Gly Ile Arg Asn
            180                 185                 190

Lys Asp Ser Arg Asn Thr Leu Asn Leu Gly Val Gly Ile Arg Thr Leu
        195                 200                 205

Glu Asn Gly Trp Leu Tyr Gly Leu Asn Thr Phe Tyr Asp Asn Asp Leu
    210                 215                 220

Thr Gly His Asn His Arg Ile Gly Leu Gly Ala Glu Ala Trp Thr Asp
225                 230                 235                 240

Tyr Leu Gln Leu Ala Ala Asn Gly Tyr Phe Arg Leu Asn Gly Trp His
                245                 250                 255

Ser Ser Arg Asp Phe Ser Asp Tyr Lys Glu Arg Pro Ala Thr Gly Gly
            260                 265                 270

Asp Leu Arg Ala Asn Ala Tyr Leu Pro Ala Leu Pro Gln Leu Gly Gly
        275                 280                 285

Lys Leu Met Tyr Glu Gln Tyr Thr Gly Glu Arg Val Ala Leu Phe Gly
    290                 295                 300

Lys Asp Asn Leu Gln Arg Asn Pro Tyr Ala Val Thr Ala Gly Ile Asn
305                 310                 315                 320

Tyr Thr Pro Val Pro Leu Leu Thr Val Gly Val Asp Gln Arg Met Gly
                325                 330                 335

Lys Ser Ser Lys His Glu Thr Gln Trp Asn Leu Gln Met Asn Tyr Arg
            340                 345                 350

Leu Gly Glu Ser Phe Gln Ser Gln Leu Ser Pro Ser Ala Val Ala Gly
        355                 360                 365

Thr Arg Leu Leu Ala Glu Ser Arg Tyr Asn Leu Val Asp Arg Asn Asn
    370                 375                 380

Asn Ile Val Leu Glu Tyr Gln Lys Gln Gln Val Val Lys Leu Thr Leu
385                 390                 395                 400

Ser Pro Ala Thr Ile Ser Gly Leu Pro Gly Gln Val Tyr Gln Val Asn
                405                 410                 415

Ala Gln Val Gln Gly Ala Ser Ala Val Arg Glu Ile Val Trp Ser Asp
            420                 425                 430

Ala Glu Leu Ile Ala Ala Gly Gly Thr Leu Thr Pro Leu Ser Thr Thr
        435                 440                 445

Gln Phe Asn Leu Val Leu Pro Pro Tyr Lys Arg Thr Ala Gln Val Ser
    450                 455                 460

Arg Val Thr Asp Asp Leu Thr Ala Asn Phe Tyr Ser Leu Ser Ala Leu
465                 470                 475                 480

Ala Val Asp His Gln Gly Asn Arg Ser Asn Ser Phe Thr Leu Ser Val
                485                 490                 495

Thr Val Gln Gln Pro Gln Leu Thr Leu Thr Ala Ala Val Ile Gly Asp
            500                 505                 510

Gly Ala Pro Ala Asn Gly Lys Thr Ala Ile Thr Val Glu Phe Thr Val
        515                 520                 525

Ala Asp Phe Glu Gly Lys Pro Leu Ala Gly Gln Glu Val Val Ile Thr
    530                 535                 540

Thr Asn Asn Gly Ala Leu Pro Asn Lys Ile Thr Glu Lys Thr Asp Ala
545                 550                 555                 560

Asn Gly Val Ala Arg Ile Ala Leu Thr Asn Thr Thr Asp Gly Val Thr
                565                 570                 575

Val Val Thr Ala Glu Val Glu Gly Gln Arg Gln Ser Val Asp Thr His
            580                 585                 590

Phe Val Lys Gly Thr Ile Ala Ala Asp Lys Ser Thr Leu Ala Ala Val
        595                 600                 605

Pro Thr Ser Ile Ile Ala Asp Gly Leu Met Ala Ser Thr Ile Thr Leu
    610                 615                 620

Glu Leu Lys Asp Thr Tyr Gly Asp Pro Gln Ala Gly Ala Asn Val Ala
625                 630                 635                 640

Phe Asp Thr Thr Leu Gly Asn Met Gly Val Ile Thr Asp His Asn Asp
                645                 650                 655

Gly Thr Tyr Ser Ala Pro Leu Thr Ser Thr Thr Leu Gly Val Ala Thr
            660                 665                 670

Val Thr Val Lys Val Asp Gly Ala Ala Phe Ser Val Pro Ser Val Thr
        675                 680                 685

Val Asn Phe Thr Ala Asp Pro Ile Pro Asp Ala Gly Arg Ser Ser Phe
    690                 695                 700

Thr Val Ser Thr Pro Asp Ile Leu Ala Asp Gly Thr Met Ser Ser Thr
705                 710                 715                 720

Leu Ser Phe Val Pro Val Asp Lys Asn Gly His Phe Ile Ser Gly Met
                725                 730                 735

Gln Gly Leu Ser Phe Thr Gln Asn Gly Val Pro Val Ser Ile Ser Pro
            740                 745                 750

Ile Thr Glu Gln Pro Asp Ser Tyr Thr Ala Thr Val Val Gly Asn Ser
        755                 760                 765

Val Gly Asp Val Thr Ile Thr Pro Gln Val Asp Thr Leu Ile Leu Ser
    770                 775                 780

Thr Leu Gln Lys Lys Ile Ser Leu Phe Pro Val Pro Thr Leu Thr Gly
785                 790                 795                 800

Ile Leu Val Asn Gly Gln Asn Phe Ala Thr Asp Lys Gly Phe Pro Lys
                805                 810                 815

Thr Ile Phe Lys Asn Ala Thr Phe Gln Leu Gln Met Asp Asn Asp Val
            820                 825                 830

Ala Asn Asn Thr Gln Tyr Glu Trp Ser Ser Ser Phe Thr Pro Asn Val
        835                 840                 845

Ser Val Asn Asp Gln Gly Gln Val Thr Ile Thr Tyr Gln Thr Tyr Ser
    850                 855                 860

Glu Val Ala Val Thr Ala Lys Ser Lys Lys Phe Pro Ser Tyr Ser Val
865                 870                 875                 880

Ser Tyr Arg Phe Tyr Pro Asn Arg Trp Ile Tyr Asp Gly Gly Arg Ser
                885                 890                 895

Leu Val Ser Ser Leu Glu Ala Ser Arg Gln Cys Gln Gly Ser Asp Met
            900                 905                 910

Ser Ala Val Leu Glu Ser Ser Arg Ala Thr Asn Gly Thr Arg Ala Pro
        915                 920                 925

Asp Gly Thr Leu Trp Gly Glu Trp Gly Ser Leu Thr Ala Tyr Ser Ser

930                 935                 940

Asp Trp Gln Ser Gly Glu Tyr Trp Val Lys Lys Thr Ser Thr Asp Phe
945                 950                 955                 960

Glu Thr Met Asn Met Asp Thr Gly Ala Leu Gln Pro Gly Pro Ala Tyr
                965                 970                 975

Leu Ala Phe Pro Leu Cys Ala Leu Ser Ile
            980                 985

(2) INFORMATION FOR SEQ ID NO: 2:

    (i) SEQUENCE CHARACTERISTICS:
        (A) LENGTH: 625 amino acids
        (B) TYPE: amino acid
        (C) STRANDEDNESS:
        (D) TOPOLOGY: linear

    (ii) MOLECULE TYPE: protein

    (xi) SEQUENCE DESCRIPTION: SEQ ID NO: 2:

Met Met Val Phe Gln Pro Ile Ser Glu Phe Leu Leu Ile Arg Asn Ala
1               5                   10                  15

Gly Met Ser Met Tyr Phe Asn Lys Ile Ile Ser Phe Asn Ile Ile Ser
            20                  25                  30

Arg Ile Val Ile Cys Ile Phe Leu Ile Cys Gly Met Phe Met Ala Gly
        35                  40                  45

Ala Ser Glu Lys Tyr Asp Ala Asn Ala Pro Gln Gln Val Gln Pro Tyr
    50                  55                  60

Ser Val Ser Ser Ser Ala Phe Glu Asn Leu His Pro Asn Asn Glu Met
65                  70                  75                  80

Glu Ser Ser Ile Asn Pro Phe Ser Ala Ser Asp Thr Glu Arg Asn Ala
                85                  90                  95

Ala Ile Ile Asp Arg Ala Asn Lys Glu Gln Glu Thr Glu Ala Val Asn
            100                 105                 110

Lys Met Ile Ser Thr Gly Ala Arg Leu Ala Ala Ser Gly Arg Ala Ser
        115                 120                 125

Asp Val Ala His Ser Met Val Gly Asp Ala Val Asn Gln Glu Ile Lys
    130                 135                 140

Gln Trp Leu Asn Arg Phe Gly Thr Ala Gln Val Asn Leu Asn Phe Asp
145                 150                 155                 160

Lys Asn Phe Ser Leu Lys Glu Ser Ser Leu Asp Trp Leu Ala Pro Trp
                165                 170                 175

Tyr Asp Ser Ala Ser Phe Leu Phe Phe Ser Gln Leu Gly Ile Arg Asn
            180                 185                 190

Lys Asp Ser Arg Asn Thr Leu Asn Leu Gly Val Gly Ile Arg Thr Leu
        195                 200                 205

Glu Asn Gly Trp Leu Tyr Gly Leu Asn Thr Phe Tyr Asp Asn Asp Leu
    210                 215                 220

Thr Gly His Asn His Arg Ile Gly Leu Gly Ala Glu Ala Trp Thr Asp
225                 230                 235                 240

Tyr Leu Gln Leu Ala Ala Asn Gly Tyr Phe Arg Leu Asn Gly Trp His
                245                 250                 255

Ser Ser Arg Asp Phe Ser Asp Tyr Lys Glu Arg Pro Ala Thr Gly Gly
            260                 265                 270

Asp Leu Arg Ala Asn Ala Tyr Leu Pro Ala Leu Pro Gln Leu Gly Gly
        275                 280                 285

Lys Leu Met Tyr Glu Gln Tyr Thr Gly Glu Arg Val Ala Leu Phe Gly
    290                 295                 300

Lys Asp Asn Leu Gln Arg Asn Pro Tyr Ala Val Thr Ala Gly Ile Asn
305                 310                 315                 320

Tyr Thr Pro Val Pro Leu Leu Thr Val Gly Val Asp Gln Arg Met Gly
                325                 330                 335

Lys Ser Ser Lys His Glu Thr Gln Trp Asn Leu Gln Met Asn Tyr Arg
            340                 345                 350

Leu Gly Glu Ser Phe Gln Ser Gln Leu Ser Pro Ser Ala Val Ala Gly
        355                 360                 365

Thr Arg Leu Leu Ala Glu Ser Arg Tyr Asn Leu Val Asp Arg Asn Asn
    370                 375                 380

Asn Ile Val Leu Glu Tyr Gln Lys Gln Gln Val Val Lys Leu Thr Leu
385                 390                 395                 400

Ser Pro Ala Thr Ile Ser Gly Leu Pro Gly Gln Val Tyr Gln Val Asn
                405                 410                 415

Ala Gln Val Gln Gly Ala Ser Ala Val Arg Glu Ile Val Trp Ser Asp
            420                 425                 430

Ala Glu Leu Ile Ala Ala Gly Gly Thr Leu Thr Pro Leu Ser Thr Thr
        435                 440                 445

Gln Phe Asn Leu Val Leu Pro Pro Tyr Lys Arg Thr Ala Gln Val Ser
    450                 455                 460

Arg Val Thr Asp Asp Leu Thr Ala Asn Phe Tyr Ser Leu Ser Ala Leu
465                 470                 475                 480

Ala Val Asp His Gln Gly Asn Arg Ser Asn Ser Phe Thr Leu Ser Val
                485                 490                 495

Thr Val Gln Gln Pro Gln Leu Thr Leu Thr Ala Ala Val Ile Gly Asp
            500                 505                 510

Gly Ala Pro Ala Asn Gly Lys Thr Ala Ile Thr Val Glu Phe Thr Val
        515                 520                 525

Ala Asp Phe Glu Gly Lys Pro Leu Ala Gly Gln Glu Val Val Ile Thr
    530                 535                 540

Thr Asn Asn Gly Ala Leu Pro Asn Lys Ile Thr Glu Lys Thr Asp Ala
545                 550                 555                 560

Asn Gly Val Ala Arg Ile Ala Leu Thr Asn Thr Thr Asp Gly Val Thr
                565                 570                 575

Val Val Thr Ala Glu Val Glu Gly Gln Arg Gln Ser Val Asp Thr His
            580                 585                 590

Phe Val Lys Gly Thr Ile Ala Ala Asp Lys Ser Thr Leu Ala Ala Val
        595                 600                 605

Pro Thr Ser Ile Ile Ala Asp Gly Leu Met Ala Ser Thr Ile Thr Leu
    610                 615                 620

Glu
625

(2) INFORMATION FOR SEQ ID NO: 3:

    (i) SEQUENCE CHARACTERISTICS:
        (A) LENGTH: 1875 base pairs
        (B) TYPE: nucleic acid
        (C) STRANDEDNESS: double
        (D) TOPOLOGY: linear

    (xi) SEQUENCE DESCRIPTION: SEQ ID NO: 3:

ATGATGGTTT TCCAGCCAAT CAGTGAGTTT CTCTTGATAA GGAATGCGGG AATGTCTATG      60

TATTTTAATA AAATAATTTC ATTTAATATT ATTTCACGAA TAGTTATTTG TATCTTTTTG     120

ATATGTGGAA TGTTCATGGC TGGGGCTTCA GAAAAATATG ATGCTAACGC ACCGCAACAG     180

GTCCAGCCTT ATTCTGTCTC TTCATCTGCA TTTGAAAATC TCCATCCTAA TAATGAAATG 240
GAGAGTTCAA TCAATCCCTT TTCCGCATCG GATACAGAAA GAAATGCTGC AATAATAGAT 300
CGCGCCAATA AGGAGCAGGA GACTGAAGCG GTGAATAAGA TGATAAGCAC CGGGGCCAGG 360
TTAGCTGCAT CAGGCAGGGC ATCTGATGTT GCTCACTCAA TGGTGGGCGA TGCGGTTAAT 420
CAAGAAATCA AACAGTGGTT AAATCGATTC GGTACGGCTC AAGTTAATCT GAATTTTGAC 480
AAAAATTTTT CGCTAAAAGA AAGCTCTCTT GATTGGCTGG CTCCTTGGTA TGACTCTGCT 540
TCATTCCTCT TTTTTAGTCA GTTAGGTATT CGCAATAAAG ACAGCCGCAA CACACTTAAC 600
CTTGGCGTCG GGATACGTAC ATTGGAGAAC GGTTGGCTGT ACGGACTTAA TACTTTTTAT 660
GATAATGATT TGACCGGCCA CAACCACCGT ATCGGTCTTG GTGCCGAGGC CTGGACCGAT 720
TATTTACAGT TGGCTGCCAA TGGGTATTTT CGCCTCAATG GATGGCACTC GTCGCGTGAT 780
TTCTCCGACT ATAAAGAGCG CCCAGCCACT GGGGGGGATT TGCGCGCGAA TGCTTATTTA 840
CCTGCACTCC CACAACTGGG GGGGAAGTTG ATGTATGAGC AATACACCGG TGAGCGTGTT 900
GCTTTATTTG GTAAAGATAA TCTGCAACGC AACCCTTATG CCGTGACTGC CGGGATCAAT 960
TACACCCCCG TGCCTCTACT CACTGTCGGG GTAGATCAGC GTATGGGGAA AAGCAGTAAG 1020
CATGAAACAC AGTGGAACCT CCAAATGAAC TATCGCCTGG GCGAGAGTTT TCAGTCGCAA 1080
CTTAGCCCTT CAGCGGTGGC AGGAACACGT CTACTGGCGG AGAGCCGCTA TAACCTTGTC 1140
GATCGTAACA ATAATATCGT GTTGGAGTAT CAGAAACAGC AGGTGGTTAA ACTGACATTA 1200
TCGCCAGCAA CTATCTCCGG CCTGCCGGGT CAGGTTTATC AGGTGAACGC ACAAGTACAA 1260
GGGGCATCTG CTGTAAGGGA AATTGTCTGG AGTGATGCCG AACTGATTGC CGCTGGCGGC 1320
ACATTAACAC CACTGAGTAC CACACAATTC AACTTGGTTT TACCGCCTTA TAAACGCACA 1380
GCACAAGTGA GTCGGGTAAC GGACGACCTG ACAGCCAACT TTTATTCGCT TAGTGCGCTC 1440
GCGGTTGATC ACCAAGGAAA CCGATCTAAC TCATTCACAT TGAGCGTCAC CGTTCAGCAG 1500
CCTCAGTTGA CATTAACGGC GGCCGTCATT GGTGATGGCG CACCGGCTAA TGGGAAAACT 1560
GCAATCACCG TTGAGTTCAC CGTTGCTGAT TTTGAGGGGA AACCCTTAGC CGGGCAGGAG 1620
GTGGTGATAA CCACCAATAA TGGTGCGCTA CCGAATAAAA TCACGGAAAA GACAGATGCA 1680
AATGGCGTCG CGCGCATTGC ATTAACCAAT ACGACAGATG GCGTGACGGT AGTCACAGCA 1740
GAAGTGGAGG GGCAACGGCA AAGTGTTGAT ACCCACTTTG TTAAGGGTAC TATCGCGGCG 1800

GATAAATCCA CTCTGGCTGC GGTACCGACA TCTATCATCG CTGATGGTCT AATGGCTTCA 1860
ACCATCACGT TGGAG 1875